# EUROPEAN PATENT APPLICATION

(11) **EP 4 679 167 A1**
(43) Date of publication of application: **14.01.2026**
(21) Application number: 23925914.6
(22) Date of filing: 12.07.2023
(51) Int. Cl.: G02C 13/00, A61L 12/02, A01N 59/00, C25B 1/16, A61L 12/12

(54) **CONTACT LENS CLEANING AND STERILIZING METHOD USING ELECTROCHEMISTRY, AND DEVICE**

(30) Priority: 07.03.2023 CN 202310211861
(71) Applicant: Suzhou 3N Biological Technology Co., Ltd, Suzhou, Jiangsu 215000 (CN)
(72) Inventor: SUN, Bixia, Suzhou, Jiangsu 215000 (CN); SUN, Ruichun, Suzhou, Jiangsu 215000 (CN); WANG, Xueyang, Suzhou, Jiangsu 215000 (CN)
(74) Representative: Gille Hrabal Partnerschaftsgesellschaft mbB Patentanwälte
(86) International application number: PCT/CN2023/106978
(87) International publication number: WO 2024/183209

(57) **Abstract**

A method and a device for cleaning and sterilizing contact lens (5) using electrochemistry, The method comprises: pouring normal saline into care chambers (1) and an auxiliary chamber (10), a cation exchange membrane (101) being provided between the care chamber (10), first electrodes (1) and the auxiliary chambers (1) and second electrodes (12) being provided inside the care chambers (1), third electrodes (102) being provided inside the auxiliary chamber (10), and contact lenses (5) being provided inside the care chambers (1); connecting the first electrodes (11) serving as a cathode and the third electrodes (102) serving as an anode to a power supply, such that the first electrodes (11) allows OH-to be generated inside the care chambers (1), and the cation exchange membrane (101) stops the OH-generated in the care chambers (1) from entering the auxiliary chamber (10); and then disconnecting the power supply, and connecting the power supply to the first electrodes (11) and the second electrodes (12), such that the anode generates ClO-, the ClO-sterilizes the contact lenses (5) and degrades denatured proteins and, under the action of an electric field force, the degraded denatured proteins move towards the electrode carrying opposite charges to those of themselves. A neutral NaCl solution is used as a base care solution, and the p H value of the solution is automatically adjusted by means of a special structure and a special method, thereby achieving high flexibility, and good sterilization and protein removal effects. A neutral NaCl solution is used as the care solution, and the pH value of the solution is automatically adjusted through a special structure and method, which provides high flexibility and excellent sterilization and protein removal effects.

## Description

### BACKGROUND OF THE INVENTION

### 1. Technical Field

The present invention relates to the field of contact lens cleaning and sterilization, in particular to a contact lens cleaning and sterilizing method using electrochemistry, and device.

### 2. Description of Related Art

Currently, contact lens care generally involves manual scrubbing with care solutions. The pH value of these care solutions tends to be acidic or alkaline, mostly alkaline. These acidic or alkaline care solutions can cause varying degrees of harm to the human body, fingers, and contact lenses. Alternatively, some use automatic care devices for cleaning and sterilizing contact lenses. Experiments have shown that if a neutral care solution is used, the cleaning and care effects are generally mediocre. To achieve excellent care results, alkaline care solutions are typically employed. However, such alkaline care solutions require specialized preparation, imposing certain cost pressures on users. Moreover, the pH value of alkaline care solutions cannot be flexibly adjusted as needed, resulting in poor flexibility. Additionally, the storage and use of alkaline care solutions present significant inconveniences. Therefore, how to use safe, harmless, readily available, and inexpensive care solutions while achieving excellent cleaning and sterilization effects has become an urgent problem for industry researchers to solve.

Additionally, current care solutions or conventional sterilization and protein removal devices lack specific protective measures, which can easily lead to discoloration or partial discoloration of contact lenses. This makes it difficult to distinguish between left and right lenses or affects their aesthetic appearance, causing inconvenience to users and impairing the normal use of contact lenses. The technical patents previously filed by the applicant regarding contact lens care, such as application numbers: 202011505217X, 2020107601353, and 2020116347313, have addressed some care issues to a certain extent. However, they still have shortcomings. Moreover, the adjustment of the pH value of the care solution employs the conventional method of adding alkali to the solution, which requires specialized preparation of alkaline care solutions, resulting in low compatibility and high costs.

Additionally, if care solutions are used to clean contact lenses and the lenses are worn directly without rinsing, it can cause harm to the eyes.

### BRIEF SUMMARY OF THE INVENTION

To address at least one of the technical problems in the prior art, the present invention provides a technical solution for a contact lens cleaning and sterilizing method using electrochemistry, and device. This solution uses neutral physiological saline as the care solution, automatically adjusts the pH value of the solution through a special structure and method, and offers high flexibility, excellent safety, and effective sterilization and protein removal. The specific solution is as follows:

In one aspect, the present invention provides a contact lens cleaning and sterilizing method using electrochemistry, which specifically includes the following steps:

S1: Pour a chloride ion-containing solution into the care chamber and auxiliary chamber, which are interconnected. A cation exchange membrane is placed between the care chamber and auxiliary chamber. The care chamber is equipped with at least a first electrode and a second electrode, while the auxiliary chamber is equipped with a third electrode. The contact lens is placed in the chloride ion-containing solution within the care chamber.

S2: Connect the first electrode and third electrode to a power source, where the first electrode serves as the cathode and the third electrode as the anode. The first electrode undergoes a reduction reaction, electrolyzing H₂O in the chloride ion-containing solution in the care chamber to produce H₂ and OH⁻. The third electrode undergoes an oxidation reaction, electrolyzing Cl⁻ in the chloride ion-containing solution in the auxiliary chamber to produce Cl₂, which dissolves in the solution to form HCl and HClO. The cation exchange membrane prevents Cl⁻ and ClO⁻ in the auxiliary chamber from entering the care chamber.

S3: When the pH value in the care chamber reaches between 8-12, disconnect the power supply to the first and third electrodes, and connect the power supply to the first and second electrodes. One of the first and second electrodes serves as the anode, and the other as the cathode. The cathode electrolyzes H₂O in the solution to produce H₂ and OH⁻, while the anode electrolyzes Cl⁻ in the solution to produce Cl₂, which dissolves to form Cl⁻ and ClO⁻. The ClO⁻ can sterilize the contact lens and degrade denatured proteins and some non-denatured proteins on the lens. The degraded denatured proteins and some non-denatured proteins move toward the electrode with the opposite charge under the electric field force formed between the anode and cathode. Non-degraded non-denatured proteins also move toward the electrode with the opposite charge under the electric field force, achieving sterilization and protein removal for the contact lens.

As a preferred embodiment of the electrochemical method for cleaning and sterilizing contact lens described in this patent, the chlorine ion-containing solution is a NaCl solution with a pH value of 7;

The first electrode, second electrode, and third electrode are all chlorine evolution electrodes.

As a preferred embodiment of the electrochemical method for cleaning and sterilizing contact lens described in this patent, in step S3, after the first electrode and second electrode are connected to the power supply, they switch between cathode and anode at certain time intervals.

As a preferred embodiment of the electrochemical method for cleaning and sterilizing contact lens described in this patent, the NaCl solution is a 0.9% mass concentration physiological saline;

In step S2, the starting voltage for connecting the first electrode and third electrode to the power supply is not less than 1.1V, the stable voltage after connection is 3.5-6.5V, and the power is disconnected after 3-11 minutes of reaction;

In step S3, the starting voltage for connecting the first electrode and second electrode to the power supply is not less than 1.1V, the stable voltage after connection is 3.5-6.5V, the pH value of the solution in the care chamber is controlled within the range of 8-12, the mass concentration of ClO⁻ in the solution is controlled at 0.1%-0.4%, and the cleaning and sterilization of the contact lens are completed after 10-60 minutes of reaction.

As a preferred embodiment of the electrochemical method for cleaning and sterilizing contact lens described in this patent, the first electrode and second electrode switch between cathode and anode every 0.5-2 minutes.

As a preferred embodiment of the electrochemical method for cleaning and sterilizing contact lens described in this patent, in step S3, the bottom heights of the first electrode and second electrode are both higher than the bottom height of the care chamber, the contact lens is placed horizontally in the chlorine ion-containing solution in the care chamber, the top height of the contact lens is lower than the bottom height of the probe, and the distance from the top of the contact lens to the liquid surface of the chlorine ion-containing solution is not less than 2mm; or

The contact lens is placed vertically in the chlorine ion-containing solution in the care chamber, the distance from the contact lens to the anode is not less than 4mm, and the distance from the contact lens to the liquid surface of the chlorine ion-containing solution is not less than 2mm.

On the other hand, the present invention provides a contact lens cleaning and sterilizing method using electrochemistryy, specifically comprising the following steps:

S1: Pour a chloride ion-containing solution into the care chamber and auxiliary chamber, which are interconnected. A cation exchange membrane is placed between the care chamber and auxiliary chamber. The care chamber is equipped with at least a first electrode and a second electrode, while the auxiliary chamber is equipped with a third electrode. The contact lens is placed in the chloride ion-containing solution within the care chamber.

S2: Connect the first electrode and third electrode to a power source, where the first electrode serves as the cathode and the third electrode as the anode. The third electrode undergoes oxidation, electrolyzing Cl⁻ in the auxiliary chamber's chloride ion-containing solution into Cl₂, which dissolves to form HCl and HClO. The first electrode undergoes reduction, electrolyzing H₂O in the care chamber's chloride ion-containing solution to produce H₂ and OH⁻, resulting in a solution containing NaCl and NaOH in the care chamber. The cation exchange membrane prevents OH⁻ generated in the care chamber from entering the auxiliary chamber, maintaining the pH environment in the care chamber.

S3: When the pH in the care chamber reaches 8-12, disconnect the power to the first and third electrodes and connect the first and second electrodes to the power source, with one serving as the anode and the other as the cathode. The cathode electrolyzes H₂O in the solution to produce H₂ and OH⁻, while the anode electrolyzes Cl⁻ into Cl₂, which dissolves to form Cl⁻ and ClO⁻. The ClO⁻ sterilizes the contact lens and degrades denatured proteins on it. The degraded proteins move toward the oppositely charged electrode under the electric field formed between the anode and cathode, achieving sterilization and protein removal.

In another aspect, the invention provides an electrochemical device for cleaning and sterilizing contact lens, comprising a power source, control switch, care chamber, and auxiliary chamber. The care and auxiliary chambers are interconnected, with a cation exchange membrane between them to block OH⁻ migration from the care chamber to the auxiliary chamber.

The care chamber contains at least a first and second electrode, which, when connected to the power source, function as anode and cathode respectively. The auxiliary chamber houses a third electrode serving as the anode.

As a preferred embodiment of the electrochemical device for cleaning and sterilizing contact lens, when a chloride ion-containing solution is added to the care chamber, the anode undergoes oxidation while the cathode undergoes reduction. The cathode electrolyzes H₂O to produce H₂ and OH⁻, and the anode electrolyzes Cl⁻ into Cl₂, which dissolves to form HCl and HClO. The ClO⁻ sterilizes the lens and degrades both denatured and some undenatured proteins, which then migrate toward oppositely charged electrodes under the electric field for sterilization and protein removal.

As a preferred embodiment, the device may include two care chambers, both connected to the auxiliary chamber or each to its own auxiliary chamber.

As a preferred embodiment, the care chamber comprises a housing groove and a sunken groove below it, with the first and second electrodes positioned in the housing groove.

As a preferred embodiment, a horizontal lens clamp is included to secure the contact lens in the sunken groove filled with chloride ion-containing solution, ensuring the lens top is at least 2mm below the solution surface.

As a preferred embodiment, a vertical lens clamp is included to secure the contact lens within the care chamber.

The distance between the contact lens and the anode in the care chamber is not less than 4 mm, and the distance between the top of the contact lens and the liquid surface of the chloride ion-containing solution is not less than 2 mm.

As a preferred embodiment of the electrochemical device for cleaning and sterilizing contact lens described in this patent, it further comprises a control system, wherein the control system is provided with an electrode switching module, and the electrode switching module is capable of switching the first electrode and the second electrode between the cathode and the anode through circuit control.

As a preferred embodiment of the electrochemical device for cleaning and sterilizing contact lens described in this patent, the first electrode and the second electrode are symmetrically arranged in the care chamber, and both the first electrode and the second electrode are close to the side wall of the care chamber.

As a preferred embodiment of the electrochemical device for cleaning and sterilizing contact lens described in this patent, the care chamber and the auxiliary chamber constitute a care assembly, and the care assembly is circular.

As a preferred embodiment of the electrochemical device for cleaning and sterilizing contact lens described in this patent, the care chamber is provided with one or more first electrodes and one or more second electrodes, and the auxiliary chamber is provided with one or more third electrodes.

As a preferred embodiment of the electrochemical device for cleaning and sterilizing contact lens described in this patent, the number of the first electrodes, the second electrodes, and the third electrodes is the same.

As a preferred embodiment of the electrochemical device for cleaning and sterilizing contact lens described in this patent, the first electrode and/or the second electrode are distributed at positions close to the side wall in the care chamber.

As a preferred embodiment of the electrochemical device for cleaning and sterilizing contact lens described in this patent, a plurality of first electrodes are uniformly distributed at positions close to the side wall in the care chamber, and/or a plurality of second electrodes are uniformly distributed at positions close to the side wall in the care chamber.

As a preferred embodiment of the electrochemical device for cleaning and sterilizing contact lens described in this patent, a limiting structure is provided at the end of the first electrode and/or the second electrode and/or the third electrode close to the bottom, and the limiting structure is fixedly accommodated at the bottom of the care chamber or the auxiliary chamber.

As a preferred embodiment of the electrochemical device for cleaning and sterilizing contact lens described in this patent, a baffle is further provided, and the baffle at least partially covers the upper part of the auxiliary chamber.

As a preferred embodiment of the electrochemical device for cleaning and sterilizing contact lens described in this patent, it further comprises a base, wherein the base is provided with a placement groove and a rinsing chamber, the care chamber and the auxiliary chamber are combined to form a care assembly, and the care assembly is placed in the placement groove;

The rinsing chamber is configured to rinse residual liquid from the contact lens after cleaning and care in the care chamber; the rinsing chamber is further provided with a flexible net, and a lens holder holding the contact lens is placed within the flexible net.

On the other hand, an electrochemical device for cleaning and sterilizing contact lens provided by this patent comprises a power source and a control switch, and further comprises a care chamber and an auxiliary chamber, wherein the auxiliary chamber is arranged around the care chamber, and a cation exchange membrane is provided between the care chamber and the auxiliary chamber, the cation exchange membrane being capable of blocking OH⁻ generated in the care chamber from entering the auxiliary chamber;

The care chamber is provided with at least a first electrode and a second electrode, wherein after the first electrode and the second electrode are connected to the power source, one of the first electrode and the second electrode serves as an anode and the other as a cathode, and the auxiliary chamber is provided with a third electrode, wherein after the third electrode and the cathode in the care chamber are connected to the power source, the third electrode serves as an anode.

As a preferred embodiment of the electrochemical cleaning and sterilization device described in this patent, the care chamber and the auxiliary chamber constitute a care assembly, and the care assembly is circular.

As a preferred embodiment of the electrochemical device for cleaning and sterilizing contact lens described in this patent, the care chamber is provided with one or more first electrodes and one or more second electrodes, and the auxiliary chamber is provided with one or more third electrodes.

As a preferred embodiment of the electrochemical device for cleaning and sterilizing contact lens described in this patent, the number of first electrodes, second electrodes, and third electrodes is the same.

As a preferred embodiment of the electrochemical device for cleaning and sterilizing contact lens described in this patent, the first electrode and/or the second electrode are distributed at positions close to the side wall in the care chamber.

As a preferred embodiment of the electrochemical device for cleaning and sterilizing contact lens described in this patent, a plurality of first electrodes are uniformly distributed at positions close to the side wall in the care chamber, and/or a plurality of second electrodes are uniformly distributed at positions close to the side wall in the care chamber.

As a preferred embodiment of the electrochemical device for cleaning and sterilizing contact lens described in this patent, a limiting structure is provided at the end of the first electrode and/or the second electrode and/or the third electrode close to the bottom, and the limiting structure is fixedly accommodated at the bottom of the care chamber or the auxiliary chamber.

As a preferred embodiment of the electrochemical device for cleaning and sterilizing contact lens described in this patent, a baffle is further provided, and the baffle at least partially covers the upper part of the auxiliary chamber.

As a preferred embodiment of the electrochemical device for cleaning and sterilizing contact lens described in this patent, it further comprises a base, wherein the base is provided with a placement groove and a rinsing chamber, the care chamber and the auxiliary chamber are combined to form a care assembly, and the care assembly is placed in the placement groove;

The rinsing chamber is configured to rinse residual liquid from the contact lens after cleaning and care in the care chamber; the rinsing chamber is further provided with a flexible net, and a lens holder holding the contact lens is placed within the flexible net.

In another aspect, this patent provides an electrochemical device for cleaning and sterilizing contact lens, comprising a power supply and a control switch, as well as a care chamber and an auxiliary chamber. A cation exchange membrane is arranged between the care chamber and the auxiliary chamber, which can prevent OH⁻ generated in the care chamber from entering the auxiliary chamber.

Multiple first electrodes and multiple second electrodes are arranged in the care chamber. When the first and second electrodes are connected to the power supply, one of them serves as an anode and the other as a cathode.

Multiple third electrodes are arranged in the auxiliary chamber. When the third electrodes are connected to the cathode in the care chamber via the power supply, the third electrodes serve as anodes.

As a preferred embodiment of the electrochemical device for cleaning and sterilizing contact lens described in this patent, the auxiliary chamber is arranged around the care chamber, with the care chamber and the auxiliary chamber corresponding one-to-one.

As a preferred embodiment of the electrochemical device for cleaning and sterilizing contact lens described in this patent, there are two care chambers, both connected to the auxiliary chamber via the cation exchange membrane.

As a preferred embodiment of the electrochemical device for cleaning and sterilizing contact lens described in this patent, the care chamber and the auxiliary chamber constitute a care assembly, and the care assembly is circular.

As a preferred embodiment of the electrochemical device for cleaning and sterilizing contact lens described in this patent, the care chamber is provided with one or more first electrodes and one or more second electrodes, and the auxiliary chamber is provided with one or more third electrodes.

As a preferred embodiment of the electrochemical device for cleaning and sterilizing contact lens described in this patent, the first electrode and/or the second electrode are distributed at positions close to the side wall in the care chamber.

As a preferred embodiment of the electrochemical device for cleaning and sterilizing contact lens described in this patent, multiple first electrodes and/or multiple second electrodes are uniformly distributed near the sidewall inside the care compartment.

As a preferred embodiment of the electrochemical device for cleaning and sterilizing contact lens described in this patent, the number of first electrodes, second electrodes, and third electrodes is the same.

As a preferred embodiment of the electrochemical device for cleaning and sterilizing contact lens described in this patent, a limiting structure is provided at the end of the first electrode and/or the second electrode and/or the third electrode close to the bottom, and the limiting structure is fixedly accommodated at the bottom of the care chamber or the auxiliary chamber.

As a preferred embodiment of the electrochemical device for cleaning and sterilizing contact lens described in this patent, a baffle is further provided, and the baffle at least partially covers the upper part of the auxiliary chamber.

As a preferred embodiment of the electrochemical device for cleaning and sterilizing contact lens described in this patent, it further comprises a base, wherein the base is provided with a placement groove and a rinsing chamber, the care chamber and the auxiliary chamber are combined to form a care assembly, and the care assembly is placed in the placement groove;

The rinsing chamber is configured to rinse residual liquid from the contact lens after cleaning and care in the care chamber; the rinsing chamber is further provided with a flexible net, and a lens holder holding the contact lens is placed within the flexible net.

Compared with the prior art, the technical solution described in this patent has at least one or more of the following beneficial effects:

This patent uses a Neutral NaCl solution as the care stock solution, which is inexpensive and easy to obtain, reducing the care cost for consumers. The Neutral NaCl solution does not contain any acidic or alkaline substances, has no acid or alkali corrosiveness, no chemical hazards, and high Safety, greatly reducing the cost of production, storage, and transportation. Applicable to contact lenses, comprising but not limited to orthokeratology lenses, scleral lenses, soft contact lenses, etc., particularly suitable for orthokeratology lenses. Orthokeratology lenses are rigid gas permeable contact lenses with corneal reshaping effects, typically made of high-oxygen permeable rigid corneal contact lens materials.

This patent automatically adjusts the pH value in the solution through a special structure and method, offering high flexibility. By providing an auxiliary chamber, where the third electrode serves as the anode, and connecting it to the cathode in the care chamber, the anode generates ClO⁻ in the auxiliary chamber, while the cathode produces OH⁻ in the care chamber, making the solution in the care chamber alkaline. Thus, the auxiliary chamber functions to adjust the pH value in the care chamber, allowing the care solution to consist solely of neutral NaCl solution. This not only enhances the versatility and accessibility of the care solution but also ensures safety by eliminating acidic or alkaline substances, greatly facilitating user operation, production, storage, and transportation. Additionally, the alkaline environment in the care chamber inhibits the production of HClO, preventing lens discoloration. Most hypochlorite ions exist as NaClO, which provides gentle sterilization and protein removal without bleaching properties, thereby avoiding lens discoloration. The ClO⁻ ions in NaClO function to sterilize and degrade proteins, and in combination with electrophoresis, effectively remove proteins.

Furthermore, the presence of a cation exchange membrane prevents OH⁻ generated in the care chamber from entering the auxiliary chamber, maintaining a strongly alkaline environment in the care chamber and suppressing the production of HClO and its impact on lens discoloration.

First, one of the first and second electrodes in the care chamber is designated as the cathode, and the third electrode in the auxiliary chamber as the anode. Upon power connection, the cathode in the care chamber undergoes a reduction reaction, electrolyzing H₂O in the chloride-containing solution to produce H₂ and OH⁻, resulting in a solution pH > 7, indicating alkalinity. The anode undergoes an oxidation reaction, electrolyzing Cl⁻ in the chloride-containing solution to produce Cl₂, which dissolves in the solution to form HCl and HClO. The cation exchange membrane prevents NaOH at the first electrode from entering the auxiliary chamber, ensuring stable pH in the care chamber. After a period of reaction, OH⁻ accumulates in the care chamber, and the power is disconnected once the solution pH reaches the desired value. Next, power is connected to the first and second electrodes in the care chamber, with one serving as the cathode and the other as the anode. The cathode continues to produce OH⁻, while the anode electrolyzes Cl⁻ in the care chamber solution to produce Cl₂, which dissolves in the strongly alkaline solution to form NaClO and NaCl. The ClO⁻ ions sterilize the contact lenses and degrade denatured proteins and some undenatured proteins on the lenses. The degraded proteins and undenatured proteins move toward the oppositely charged electrode under the electric field formed between the cathode and anode, achieving sterilization and protein removal from the contact lenses.

The electrochemical cleaning and sterilization technology of this patent exhibits excellent bactericidal and protein removal effects, effectively degrading proteins and achieving instant sterilization, reaching sterilization levels within 10 minutes. The sterilization and protein removal processes do not damage the contact lens itself, with good stability and low toxicity and side effects. Using this device for automatic cleaning eliminates the need for manual scrubbing and avoids contact with care solutions, reducing harm to the human body. The sterilization and protein removal time for contact lenses is short, requiring only 10-60 minutes. Conventional care solutions take at least 2-4 hours and require manual scrubbing, being time-consuming and labor-intensive.

In the care chamber and auxiliary chamber, at least the anode is a chlorine evolution electrode. In a sodium chloride solution, the anode undergoes an oxidation reaction to release chlorine gas from the chloride ions in the solution. The chlorine evolution electrode exhibits strong corrosion resistance, excellent chlorine/acid resistance, good stability, oxidation resistance, long service life, preventing anode reactions or byproduct generation that could contaminate electrolyte or cathode products; facilitates bubble discharge between/on electrode surfaces, effectively reducing voltage in electrolytic cell; easy to shape with high precision. Common metal electrodes mainly include ruthenium, iridium, and platinum series. Other inert electrodes (e.g., graphite, graphene materials) can also serve as chlorine evolution electrodes provided material morphology stability is maintained. The electrode may also be a coated electrode, preferably using titanium or titanium alloy substrate coated with ruthenium, iridium, platinum series metal coatings. The cathode and anode are preferably symmetrically distributed on both sides of the care chamber, positioned close to sidewalls for better protein removal.

Commercially available care solutions tend to damage contact lenses with sterilization rates typically around 90%. This patent achieves instant sterilization, not only with excellent sterilization effects but also at a rapid speed, achieving a 99% bacterial kill rate and 99% fungal kill rate within 3 minutes, and reaching sterilization level by eliminating Bacillus subtilis in 10 minutes. Moreover, commercially available care solutions cannot be directly applied to the eyes. If users forget to rinse after completing contact lens care and wear the lenses directly, residual care solution on the lenses may cause eye damage. This patent, however, allows direct application to the eyes. If users forget to rinse the cleaned contact lenses before wearing them, no harm will be caused to the eyes. Additionally, AB solution contains potassium bromide, which generates liquid bromine during use. Liquid bromine can corrode the coating of contact lenses, leading to coating detachment. Coating detachment not only affects the lifespan of the contact lenses but also may cause inflammation if the detached coating enters the eyes. Furthermore, residual bromine on the contact lenses poses safety concerns.

After the first and second electrodes in the care chamber of this patent are powered, the chloride ion-containing solution can generate various oxidizing substances and free radicals, particularly hypochlorous acid and hypochlorite ions, which can disrupt protein peptide chains. The mass concentration of hypochlorite ions in this patent is preferably 0.1%, while the hypochlorite ion concentration in Solution A of AB solution is 0.375%. Although AB solution achieves good sterilization and protein removal effects, contact lenses should not be soaked in AB solution for extended periods, as it may cause damage such as discoloration and corrosion. Due to the incorporation of electrophoresis technology, this patent achieves excellent sterilization and protein removal effects with a preferred hypochlorite ion concentration of 0.1%, which is less than one-third of that in AB solution. It is safer and more convenient to use, and by appropriately controlling relevant parameters, it can reduce discoloration, avoid coating damage, and prevent corrosion of contact lenses.

In this invention, the mass concentration range of hypochlorite ions is controlled between 0.1%-0.4%. If the mass concentration range of hypochlorite ions is 0.01%-0.1%, the solution containing hypochlorite ions in this range can be directly applied to the eyes and mucous membranes, providing good sterilization effects but weaker protein removal effects. When the mass concentration range of hypochlorite ions is 0.4%-1%, the sterilization and protein removal effects are better. However, high hypochlorite ion concentration can only remain stable under strong alkaline conditions and may damage non-antioxidant materials in principle. Therefore, overall, a hypochlorite ion concentration of 0.1%-0.4% is the optimal choice.

Through extensive long-term research, investigators have identified the optimal concentration range for hypochlorite ions and determined experimental conditions that maintain the hypochlorite ion concentration within the optimal range, comprising an optimal sodium chloride solution concentration of 0.9%, a starting voltage of no less than 1.1V when the first and second electrodes are powered, and a stable voltage range of 3.5-6.5V after power is applied. When the contact lens is placed horizontally in the care compartment, the height of the top of the contact lens is lower than the bottom of the probe, and the distance from the top of the contact lens to the surface of the NaCl solution is not less than 2 mm; or when the contact lens is placed vertically in the care compartment, the distance from the contact lens to the anode is not less than 4 mm, and the distance from the contact lens to the surface of the NaCl solution is not less than 2 mm. The reaction time after power is turned on is 10-60 minutes.

The chloride ion-containing solution of this patent, under the action of an electric field, can generate hydroxyl radicals (OH•), oxygen radicals (O•), chlorine radicals (Cl•), hydrogen peroxide (H₂O₂), ozone (O₃), hypochlorite ions (ClO⁻), hypochlorous acid (HClO), chlorine gas (Cl₂), hydrogen ions (H⁺), and other substances, combined with electrophoresis under specific conditions, resulting in effective protein removal and sterilization, comparable to AB solution. Moreover, compared to AB solution, the concentration of hypochlorite ions generated in the reaction is less than one-third of that in AB solution, making it safer to use and more convenient to operate.

The present invention includes an electrode switching module. After the first electrode and the second electrode in the care compartment are powered, they switch between the cathode and anode at certain time intervals, thereby preventing the accumulation of single or single-type ions near the cathode and anode, which could lead to uneven concentration, especially the adverse effects of HClO accumulation on the contact lens, such as discoloration. Specifically, after the first electrode and the second electrode are powered, the H₂O in the chloride ion-containing solution at the cathode is electrolyzed to produce H₂ and OH⁻, e+2H₂O=H₂ and OH⁻, OH⁻ accumulates, and the pH of the solution near the cathode is greater than 7. The Cl⁻ in the chloride ion-containing solution at the anode is electrolyzed to Cl₂, which dissolves in water to produce HCl and HClO. The accumulation of HCl and HClO results in a pH of less than 7 in the anode solution. Switching the first electrode and the second electrode between the cathode and anode can neutralize HClO, converting more of it into NaClO, thereby reducing its impact on the color of the contact lens. At the same time, switching the electrodes can uniformly mix other ions or molecules generated at the cathode and anode, aiding in the uniform sterilization and protein removal from the contact lens.

The electrochemical cleaning and sterilization technology of this patent, compared to the traditional manual rubbing method for cleaning contact lenses in the market, applies the electrophoretic dissociation electric field force only to the charged particles on the contact lens, such as proteins, bacteria, and fungi, more effectively separating denatured proteins from the contact lens without damaging the lens itself. This avoids issues such as insufficient rubbing force leading to incomplete removal of deposited proteins, excessive rubbing force causing lens breakage, uneven rubbing force leading to lens deformation or scratches, and insufficient finger cleaning causing lens scratches or bacterial infections.

The electrochemical sterilization and protein removal technology of the present invention can kill microorganisms such as bacteria, and its sterilization principle mainly includes the following aspects:
1. Electrolysis produces hypochlorous acid to block the bacterial protein synthesis pathway
   Electrolysis of a chloride ion-containing solution generates hypochlorous acid, which is a strong oxidizing agent with potent oxidative capacity. The sterilization principle of hypochlorous acid is to oxidize and denature proteins in microorganisms, preventing them from normal replication and thus losing viability. Hypochlorous acid is 80 times more effective than hypochlorite ions in killing microorganisms and can generate hydroxyl radicals that act on different bacteria. Moreover, long-term use does not lead to bacterial resistance to electrolyzed physiological saline, and it has no toxic side effects on the cornea.
2. Electroincorporation: Under the influence of an electric field, the process of incorporating substances from a solution into cells is called electroincorporation. Based on the effects of an external electric field on bacterial growth, activity, metabolism, morphology, and movement, combined with hydroxyl radicals (OH•), oxygen radicals (O•), chlorine radicals (Cl•), hydrogen peroxide (H₂O₂), ozone (O₃), hypochlorite (ClO⁻), hypochlorous acid (HClO), chlorine gas (Cl₂), hydrogen ions (H⁺), a novel sterilization and protein removal technology has been developed. Applying an appropriate current can, on one hand, interfere with gene expression in cells, inhibit ATPase activity, reduce protein content within bacterial cells, affect intracellular free radical reactions and the synthesis of biological macromolecules, and suppress cell proliferation by neutralizing the negative charge on cell surfaces, ultimately leading to cell apoptosis, senescence, and death. On the other hand, it can enhance the permeability of microbial cells, causing electroincorporation, allowing hydroxyl radicals (OH•), oxygen radicals (O•), chlorine radicals (Cl•), hydrogen peroxide (H₂O₂), ozone (O₃), hypochlorite (ClO⁻), hypochlorous acid (HClO), chlorine gas (Cl₂), hydrogen ions (H⁺) to penetrate microbial cells, generating cytotoxicity, inducing structural and functional disorders in cells, achieving inactivation, sterilization, and disinfection effects.
3. Micro-electrolysis generates active substances that disrupt the organic chain structure of cells
   Under the action of a physical field, the current density is controlled in an energized state, and the excited electrons are transferred from the anode to the cathode through the medium of water. During the transfer process, oxidizing substances such as O radicals, ClO-, Cl-, OH-, H2O2, etc., can be generated. These active substances can react with any molecules of living cells, such as sugars, phospholipids, organic acids, etc., with a fast reaction rate, damaging the cell membrane and penetrating into the cell membrane to disrupt the chain structure of organic matter. They oxidize bacterial cell RNA and DNA, leading to their inactivation or death. Meanwhile, bacteria in water generally carry a negative charge and will migrate and aggregate toward the anode, which can cause direct lethal discharge to the bacteria.
4. Microcurrent punctures bacterial cell walls, rapidly oxidizing bacterial RNA/DNA.
   The current directly acts on the cell walls, causing mechanical damage to bacteria in the solution and enhancing the oxidation of bacterial and viral RNA/DNA.
5. Microcurrent breaks bacterial clusters, reducing bacterial tolerability.
   The action of current alters the relative dispersion environment of bacteria in water, significantly reducing the stability of aggregated mixtures. When bacteria and viruses are considered as a colloidal system composed of water, proteins, and nucleic acids, microcurrent reduces the stability of bacteria, thereby lowering their tolerance to hypochlorous acid.
6. Electron-activated water enhances the contact surface between hypochlorous acid and bacteria.
   The properties of water depend on the structural changes of water molecule electrons, mainly the distribution, shape, and direction of electron clouds. Studies have confirmed that electron clouds can be altered by external environments. Under low voltage and microcurrent, the four pairs of electrons in water molecules transition from low to high orbitals, increasing the electron energy level, resulting in a loss of potential energy in activated water molecules and a decrease in their potential. The potential difference between water molecules and interfaces (microbial surfaces) is reduced. This change may affect the aggregation state of bacteria/virus particles. Moreover, hydrophilic bacteria such as Streptococcus viridans, Pseudomonas aeruginosa, and Staphylococcus, commonly found on contact lenses, become more susceptible to hypochlorous acid after microcurrent treatment, improving bactericidal efficiency.

Auxiliary chambers are arranged in a one-to-one correspondence around the care chambers, with each care chamber independently controlled to avoid mutual interference between different lenses and different reaction processes. This design is more aesthetically pleasing in appearance and more intuitive in operation, facilitating user interaction, preventing misuse, and providing consumers with a better and more comfortable user experience. The auxiliary chamber is arranged around the care chamber, making full use of space with more reasonable design, effectively reducing the volume of the care assembly.

Multiple electrodes of the same polarity are arranged in the care chamber, providing high cleaning efficiency and speed, while making NaClO, NaOH, and HClO in the care chamber more uniform, resulting in more even contact lens cleaning. A potential difference is formed internally between or mutually between the first electrode pair and the second electrode pair. Multiple electrodes of the same polarity are uniformly distributed near the sidewall inside the care chamber, eliminating the need for electrode polarity switching.

The electrodes are equipped with limiting structures, which not only ensure sealed connection between the electrodes and the care assembly to prevent liquid leakage, but also firmly install the electrodes on the care assembly, reducing the likelihood of loosening or shaking during device assembly or use.

The rinsing chamber is provided with a flexible mesh, which physically cleans the surface of the contact lens during ultrasonic vibration rinsing, ensuring cleaner and more thorough surface rinsing.

Additional aspects and advantages of the invention will be set forth in part in the description that follows, and in part will be obvious from the description, or may be learned by practice of the invention.

### BRIEF DESCRIPTION OF THE SEVERAL VIEWS OF THE DRAWINGS

To describe the technical solutions of the present invention more clearly, the following briefly introduces the accompanying drawings required for describing the embodiments or the prior art. Obviously, the accompanying drawings in the following description show merely some embodiments of the present invention, and a person of ordinary skill in the art may still derive other drawings from these accompanying drawings without creative efforts.
FIG 1 is a three-dimensional schematic structural view of an electrochemical contact lens cleaning and sterilization device according to this patent;
FIG. 2 isan exploded schematic structural view of the electrochemical contact lens cleaning and disinfection device according to the present patent;
FIG. 3 is a three-dimensional schematic structural view of the care assembly according to the present patent;
FIG. 4 is an exploded schematic structural view of an embodiment of the care assembly according to the present patent;
FIG. 5 is a three-dimensional schematic structural view of the care chamber and auxiliary chamber described in FIG. 4;
FIG. 6 is a cross-sectional schematic view of another embodiment of the care assembly according to the present patent;
FIG. 7 is a three-dimensional schematic structural view of the lateral lens clamp according to the present patent;
FIG. 8 is an exploded schematic structural view of the lateral lens clamp according to the present patent;
FIG. 9 is a three-dimensional schematic structural view of the vertical lens clamp according to the present patent;
FIG. 10 is an exploded schematic structural view of the vertical lens clamp according to the present patent;
FIG. 11 is a circuit schematic view of the electrochemical contact lens cleaning and disinfection device according to the present patent;
FIG. 12 is a schematic cross-sectional structural diagram of yet another embodiment of the nursing assembly according to the present patent;
FIG. 13 is a schematic cross-sectional structural diagram of the nursing assembly shown in FIG. 12 along the transverse direction;
FIG. 14 is a schematic cross-sectional structural diagram of the nursing assembly shown in FIG. 12 along the vertical direction;
FIG. 15 is an exploded schematic structural view of one configuration of the nursing assembly shown in FIG. 12;
FIG. 16 is an exploded schematic structural view of another configuration of the nursing assembly shown in FIG. 12;
FIG. 17 is a three-dimensional schematic structural view of the profiled connector in FIG. 16 from one viewing angle;
FIG. 18 is a three-dimensional schematic structural view of the profiled connector in FIG. 16 from another viewing angle;
FIG. 19 is a three-dimensional schematic structural view of the second connector in FIG. 16;
FIG. 20 is a three-dimensional schematic structural view of the electrode in FIG. 12;
FIG. 21 is an exploded schematic structural view of another embodiment of the electrochemical device for cleaning and sterilizing contact lens according to the present patent;
FIG. 22 is a partially enlarged schematic view of section R in FIG. 21;
FIG. 23 is a three-dimensional schematic structural view of the electrochemical device for cleaning and sterilizing contact lens described in FIG. 21;
FIG. 24 is a schematic cross-sectional structural diagram of FIG. 23;
FIG. 25 is a partially enlarged schematic view of section M in FIG. 24;
FIG. 26 is an exploded schematic structural view of yet another embodiment of the electrochemical device for cleaning and sterilizing contact lens according to the present patent;
FIG. 27 is an exploded schematic structural view of the care assembly in FIG. 26;
FIG. 28 is a three-dimensional schematic structural view of the care assembly in FIG. 26;

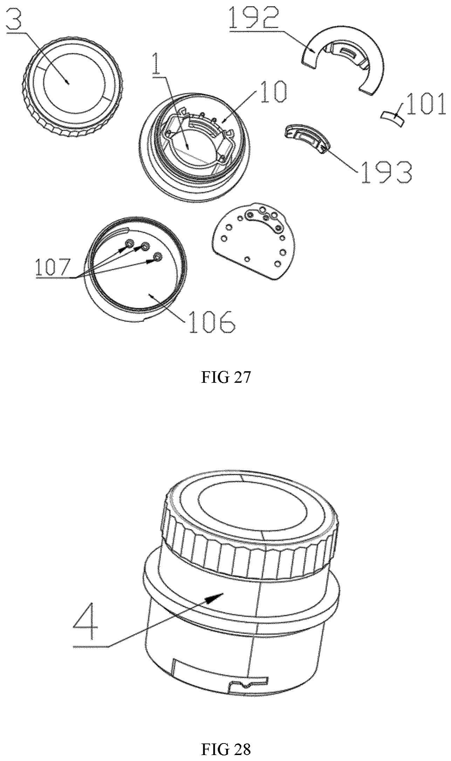

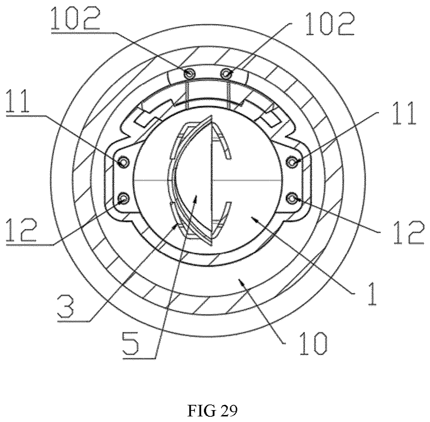

FIG. 29 is a schematic cross-sectional structural diagram of the care assembly in FIG. 28. Wherein, 1-care chamber, 10-auxiliary chamber, 2-base, 101-cation exchange membrane, 102-third electrode, 103-limit slot, 104-cover edge, 105-gap, 106-bottom cover, 107-second electrode contact, 3-lens clip, 4-care assembly, 5-contact lens, 6-PCB board, 7-control panel, 11-first electrode, 12-second electrode, 13-accommodation slot, 14-sunken slot, 16-cover body, 161-mounting port, 171-main area, 172-extension area, 18-baffle, 191-care chamber body, 192-profiled connector, 193-second connector, 194-opening slot, 195-side plate, 196-mounting slot, 197-pressing block, 198-annular protrusion, 199-second protrusion, 21-placement groove, 211-surrounding edge, 212-conductive contact, 22-rinsing chamber, 31-lateral lens clamp, 310-first cover, 311-mounting base, 312-support member, 313-limit member, 314-limit hole, 32-vertical lens clip, 320-second cover, 321-fixed base, 322-accommodation member, 323-accommodation hole.

### DETAILED DESCRIPTION OF THE INVENTION

Embodiments of the present invention are described in detail below, examples of which are illustrated in the accompanying drawings, wherein like or similar reference numerals designate like or similar elements or elements having like or similar functions throughout. The embodiments described below with reference to the accompanying drawings are exemplary and are intended to explain the present invention, but should not be construed as limiting the present invention. Based on the embodiments of the present invention, all other embodiments obtained by those of ordinary skill in the art without creative efforts shall fall within the protection scope of the present invention.

In the description of the present invention, it should be understood that the terms "upper," "lower," "top," "bottom," "inner," "outer," "front end," "rear end," "both ends," "one end," "the other end," and other indications of orientation or positional relationships are based on the orientation or positional relationships shown in the accompanying drawings, and are only for the convenience of describing the present invention and simplifying the description, rather than indicating or implying that the referred device or element must have a specific orientation, be constructed and operated in a specific orientation, and thus should not be construed as limiting the present invention. In the description of the present invention, the meaning of "a plurality" is two or more, unless otherwise explicitly and specifically defined. Furthermore, the terms "first," "second," "third," "fourth," "fifth" are used for descriptive purposes only and should not be construed as indicating or implying relative importance.

In the description of the present invention, unless otherwise expressly specified and defined, the terms "provided with," "provided," "connected," "mounted with," "sleeved," "opened," "fixed," and other terms should be understood broadly. For example, the connection may be a fixed connection, a detachable connection, or an integral connection; it may be a mechanical connection or an electrical connection; it may be a direct connection or an indirect connection through an intermediate medium, and it may be the internal communication of two elements or the interaction relationship between two elements. For those of ordinary skill in the art, the specific meanings of the above terms in the present invention can be understood according to specific circumstances.

In one aspect, the present invention provides a contact lens cleaning and sterilizing method using electrochemistry, which specifically includes the following steps:

S1: Pour a chloride ion-containing solution into the care chamber and auxiliary chamber, which are interconnected. A cation exchange membrane is placed between the care chamber and auxiliary chamber. The care chamber is equipped with at least a first electrode and a second electrode, while the auxiliary chamber is equipped with a third electrode. The contact lens is placed in the chloride ion-containing solution within the care chamber.

S2: Connect the first electrode and the third electrode to a power supply, wherein the first electrode serves as the cathode and the third electrode serves as the anode. A reduction reaction occurs at the first electrode, where the first electrode electrolyzes H₂O in the chloride ion-containing solution within the care chamber to produce H₂ and OH⁻. An oxidation reaction occurs at the third electrode, where the third electrode electrolyzes Cl⁻ in the chloride ion-containing solution within the auxiliary chamber to produce Cl₂, which dissolves in the solution to form HCl and HClO. The cation exchange membrane prevents Cl and ClO⁻ in the auxiliary chamber from entering the care chamber, thereby maintaining an alkaline pH environment in the care chamber. This ensures that during subsequent operation of the first and second electrodes in the care chamber, NaClO is easily produced while HClO is minimized, reducing the adverse effects of HClO on lens discoloration and material properties.

S3: When the pH value in the care chamber reaches between 8-12, disconnect the power supply to the first and third electrodes and connect the power supply to the first and second electrodes. One of the first and second electrodes serves as the anode, and the other serves as the cathode. The cathode electrolyzes H₂O in the solution to produce H₂ and OH⁻, while the anode electrolyzes Cl⁻ in the solution to produce Cl₂, which dissolves to form Cl and ClO⁻. The ClO⁻ can sterilize the contact lens and degrade denatured proteins and some non-denatured proteins on the lens. The degraded denatured proteins and some non-denatured proteins move toward the electrode with the opposite charge under the electric field force formed between the anode and cathode. Undegraded non-denatured proteins also move toward the electrode with the opposite charge under the electric field force, achieving sterilization and protein removal for the contact lens.

Of course, the cation exchange membrane can also prevent OH⁻ generated in the care chamber from entering the auxiliary chamber, ensuring the pH environment in the care chamber.

Preferably, the chloride ion-containing solution is an NaCl solution. Further preferably, the pH value of the NaCl solution is 7.

In a preferred embodiment, the NaCl solution is a physiological saline with a mass concentration of 0.9%.

In step S2, the starting voltage for connecting the first electrode and third electrode to the power supply is not less than 1.1V, the stable voltage after connection is 3.5-6.5V, and the power is disconnected after 3-11 minutes of reaction;

In step S3, the starting voltage for connecting the first electrode and second electrode to the power supply is not less than 1.1V, the stable voltage after connection is 3.5-6.5V, the pH value of the solution in the care chamber is controlled within the range of 8-12, the mass concentration of ClO⁻ in the solution is controlled at 0.1%-0.4%, and the cleaning and sterilization of the contact lens are completed after 10-60 minutes of reaction.

In step S2, after connecting the first and third electrodes to the power supply, the first or second electrode serves as the cathode, and the third electrode serves as the anode. The following reactions occur at the cathode and anode:
**Cathode:**

   2H₂O+e=H₂↑+2OH⁻
**Anode:**

   2Cl⁻-e→Cl₂↑

   Cl₂+H₂O=HCl+HClO

In step S3, after the first electrode and the second electrode are connected to the power supply, the following reactions occur at the cathode and anode:
**Cathode:**

   2H₂O+e=H₂↑+2OH⁻ (I)
**Anode:**

   2Cl⁻-e→Cl₂↑ (II)

   Cl₂+H₂O=HCl+HClO (III)

The resulting HClO undergoes the following reaction:

2HClO=2HCl+O₂↑ (IV)

Na⁺+ClO⁻=NaClO (V)

NaClO+H₂O=HClO+NaOH (VI)

When the chloride ion-containing solution is in a neutral environment, the solution mainly consists of a mixture of NaClO, NaOH, and HClO. When the chloride ion-containing solution is in an alkaline environment, the solution primarily contains NaClO and NaOH. Because in an alkaline environment, the dynamic equilibrium of the reversible reaction in Equation VI shifts to the left, resulting in a higher concentration of NaClO and a lower concentration of HClO. NaClO provides milder sterilization and protein removal without bleaching properties, preventing lens discoloration. The ClO⁻ ion in NaClO can sterilize and degrade proteins, and in combination with electrophoresis, it effectively removes proteins. HClO has a sterilizing effect but also a bleaching effect. A high concentration of HClO can cause lens discoloration, while a lower concentration of HClO significantly reduces its impact on the color of contact lenses.

In a preferred embodiment, in step S3, after the first electrode and the second electrode are connected to the power supply, the first electrode and the second electrode are switched between the cathode and the anode at certain time intervals. Preferably, the first electrode and the second electrode are switched between the cathode and the anode every 0.5-2 minutes.

The first electrode, second electrode, and third electrode are all chlorine evolution electrodes. In a chloride ion-containing solution, the anode undergoes an oxidation reaction to release chlorine gas from the chloride ions in the solution. The chlorine evolution electrode exhibits strong corrosion resistance, excellent chlorine/acid resistance, good stability, oxidation resistance, long service life, preventing anode reactions or byproduct generation that could contaminate electrolyte or cathode products; facilitates bubble discharge between/on electrode surfaces, effectively reducing voltage in electrolytic cell; easy to shape with high precision. Common metal electrodes mainly include ruthenium, iridium, and platinum series. Other inert electrodes (e.g., graphite, graphene materials) can also serve as chlorine evolution electrodes provided material morphology stability is maintained. The electrode substrate preferably adopts a titanium alloy substrate or a platinum-plated substrate. The cathode and anode are preferably symmetrically distributed on both sides of the care chamber, positioned close to sidewalls for better protein removal. In the example, both the first electrode and the second electrode are probes or electrode sheets, which can be adjusted as needed.

In a preferred embodiment, in step S3, the bottom heights of the first electrode and the second electrode are both higher than the bottom height of the care chamber. The contact lens is horizontally placed in the chloride ion-containing solution within the care chamber, with the top height of the contact lens lower than the bottom height of the probe, ensuring that the distance from the top of the contact lens to the liquid surface of the chloride ion-containing solution is not less than 2 mm. Alternatively, the contact lens is vertically placed in the chloride ion-containing solution within the care chamber, ensuring that the distance from the contact lens to the anode is not less than 4 mm and the distance from the contact lens to the liquid surface of the chloride ion-containing solution is not less than 2 mm. If the electrode switching module on the device is used, the contact lens is preferably positioned at the middle between the first electrode and the second electrode, with a distance of no less than 4 mm from both the first electrode and the second electrode. If the electrode switching module on the device is not used, the distance from the contact lens to the anode should be no less than 4 mm. Of course, the contact lens can also be positioned at the middle between the first electrode and the second electrode.

In a preferred embodiment, it further includes step S4;

S4: After the completion of step S3 care, rinse the contact lens with sterile physiological saline or a multi-purpose eye care solution. Of course, after rinsing, it is preferable to drop lubricant into the concave surface of the contact lens before wearing it.

On the other hand, the present patent provides an electrochemical device for cleaning and sterilizing contact lens, comprising a power supply and control switch, a care chamber 1 and an auxiliary chamber 10, wherein the care chamber 1 and the auxiliary chamber 10 are connected, and a cation exchange membrane is arranged between the care chamber 1 and the auxiliary chamber 10, said cation exchange membrane being capable of blocking anions in the auxiliary chamber 10 from entering the care chamber;

The care chamber 1 is provided with at least a first electrode 11 and a second electrode 12. When the first electrode 11 and the second electrode 12 are connected to the power supply, one of the first electrode 11 and the second electrode 12 serves as an anode and the other as a cathode. The auxiliary chamber 10 is provided with a third electrode, which serves as the anode;

The power supply can provide electrical energy to the care chamber and the auxiliary chamber, and the control switch can at least control the device to turn on or off.

Of course, the cation exchange membrane can also prevent OH⁻ generated in the care chamber from entering the auxiliary chamber, ensuring the pH environment in the care chamber.

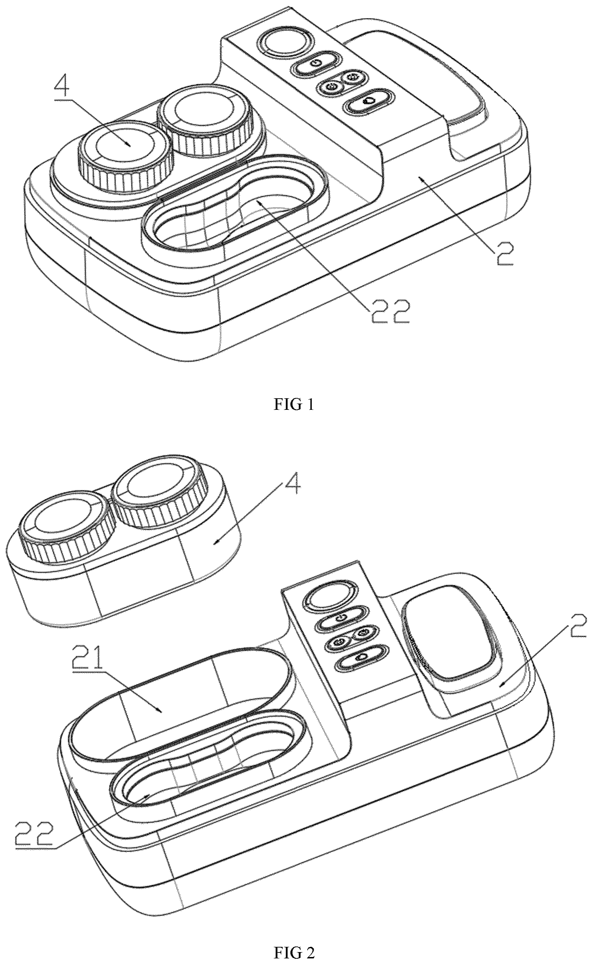

As shown in FIG. 1-10, the cleaning and disinfection device comprises a care chamber 1, an auxiliary chamber 10, a base 2, and a control system. The care chamber 1 and the auxiliary chamber 10 are connected, and a cation exchange membrane is arranged between the care chamber 1 and the auxiliary chamber 10, said cation exchange membrane being capable of blocking anions in the auxiliary chamber 10 from entering the care chamber;

The care chamber 1 is provided with at least a first electrode 11 and a second electrode 12. When the first electrode 11 and the second electrode 12 are connected to a power supply, one of the first electrode 11 and the second electrode 12 serves as an anode, and the other serves as a cathode. The auxiliary chamber 10 is provided with a third electrode 102, which serves as the anode.

Of course, the cation exchange membrane can also prevent OH⁻ generated in the care chamber from entering the auxiliary chamber, ensuring the pH environment in the care chamber.

Preferably, in a working environment where a chloride ion-containing solution is added to the care chamber, an oxidation reaction occurs at the anode, and a reduction reaction occurs at the cathode. The cathode electrolyzes H₂O in the chloride ion-containing solution to produce H₂ and OH⁻; the anode electrolyzes Cl⁻ in the chloride ion-containing solution to produce Cl₂, which dissolves in the solution to form HCl and HClO. The ClO⁻ can sterilize the contact lens and degrade denatured proteins and some non-denatured proteins on the contact lens. The degraded denatured proteins and some non-denatured proteins move toward the electrode with the opposite charge under the electric field force formed between the anode and the cathode. Undegraded non-denatured proteins also move toward the electrode with the opposite charge under the electric field force formed between the anode and the cathode, thereby achieving sterilization and protein removal for the contact lens.

Preferably, there are two care chambers 1, both of which are connected to the auxiliary chamber 10, or each of the two care chambers 1 is connected to a corresponding auxiliary chamber 10. In the example, there is one auxiliary chamber 10, and both care chambers 1 are connected to the auxiliary chamber 10. The auxiliary chamber 10 is provided with two third electrodes, each matching one of the two care chambers. A cation exchange membrane 101 is provided between the auxiliary chamber 10 and the two care chambers. In the example, the care chamber 1 and the auxiliary chamber 10 are arranged on the same housing and collectively referred to as the care assembly 4.

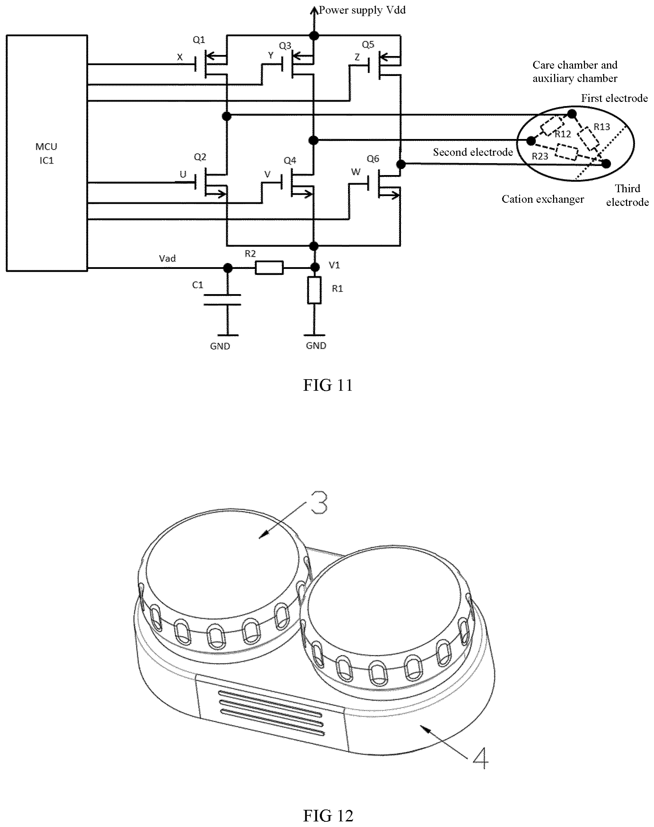

The control system can control the operation of the device. Preferably, the control system is provided with an electrode switching module. After the first electrode and the second electrode are powered, one serves as the cathode and the other as the anode. The electrode switching module can switch the first electrode and the second electrode between the cathode and the anode through circuit control. In a preferred example, the electrode switching module can switch the first electrode and the second electrode between the cathode and the anode every 0.5-2 minutes. More preferably, the first electrode and the second electrode switch between the cathode and the anode every 1 minute. FIG. 11 shows a schematic diagram of the electrode operation and switching circuit, and Table 1 below presents the driving logic diagram of the circuit:

**Table 1 Driving logic table for the first electrode, second electrode, and third electrode circuits**

| **Electrode state** | | **First state** | **Second state** | **Third state** | **Fourth state** |
|---|---|---|---|---|---|
| Drive Upper Arm | X | L | L | H | L |
| | Y | L | H | L | L |
| | Z | H | L | L | L |
| Driven lower arm | U | H | H | L | L |
| | V | L | L | H | L |
| | W | L | L | L | L |
| First electrode | | - | - | + | Floating |
| Second electrode | | Floating | + | - | Floating |
| third electrode | | + | Floating | Floating | Floating |

In the circuit schematic of FIG. 11, IC1 is the microcontroller unit MCU, Q1, Q3, and Q5 are all N-channel MOSFETs, Q2, Q4, and Q6 are all P-channel MOSFETs, R1 and R2 are resistors, Cl is a capacitor, the care chamber and auxiliary chamber contain physiological saline with a mass concentration of 0.9%, the first electrode and second electrode are arranged in the care chamber, the third electrode is arranged in the auxiliary chamber, there is a simulated electrode R12 between the first electrode and second electrode, a simulated electrode R13 between the first electrode and third electrode, and a simulated electrode R23 between the second electrode and third electrode. The level signals driven by the MCU: H is high level, L is low level.

As shown in the circuit logic driving table of Table 1, the first state: the driving upper arm X of MCU is low level (L), the driving upper arm Y is low level (L), the driving upper arm Z is high level (H), the driving lower arm U of MCU is high level (H), the driving upper arm V is low level (L), the driving lower arm W is low level (L), then the current forms a current loop from power supply Vdd→Q5→third electrode→R13→first electrode→Q2→R1. At this time, the first electrode of the care chamber is negative (-), the second electrode is floating, the third electrode is positive (+), the current flows through R1, generating a divided voltage V1, which is filtered by R2 and Cl, and then enters the MCU AD sampling voltage Vad. The voltage at this port is used to determine whether it is a current loop and whether a current loop is established. If Vad has voltage, the first electrode and the third electrode are working normally; if Vad voltage is 0V, the operation is abnormal.

In the first state, the third electrode is positive, that is, the anode. The third electrode electrolyzes Cl⁻ in the chloride ion-containing solution in the auxiliary chamber into Cl₂, which dissolves in the solution to produce HCl and HClO. The first electrode is negative, that is, the cathode. The first electrode electrolyzes H₂O in the chloride ion-containing solution in the care chamber to produce H₂ and OH⁻, thereby regulating the pH value in the care chamber. The cation exchange membrane can block ClO⁻ in the auxiliary chamber from entering the care chamber, reducing the impact of ClO⁻ on the contact lens during pH regulation in the care chamber.

Furthermore, the presence of a cation exchange membrane prevents OH⁻ generated in the care chamber from entering the auxiliary chamber, maintaining a strongly alkaline environment in the care chamber and suppressing the production of HClO and its impact on lens discoloration.

The second state: the driving upper arm X of MCU is low level (L), the driving upper arm Y is high level (H), the driving upper arm Z is low level (L), the driving lower arm U of MCU is high level (H), the driving upper arm V is low level (L), the driving lower arm W is low level (L), then the current forms a current loop from power supply Vdd→Q3→second electrode→R12→first electrode→Q2→R1. At this time, the first electrode of the care chamber is negative (-), the second electrode is positive (+), the third electrode is floating, the current flows through R1, generating a divided voltage V1, which is filtered by R2 and Cl, and then enters the MCU AD sampling voltage Vad. The voltage at this port is used to determine whether it is a current loop and whether a current loop is established. If Vad has voltage, the care chamber is working normally; if Vad voltage is 0V, the care chamber is working abnormally.

The third state: the driving upper arm X of MCU is high level (H), the driving upper arm Y is low level (L), the driving upper arm Z is low level (L), the driving lower arm U of MCU is low level (L), the driving upper arm V is high level (H), the driving lower arm W is low level (L), then the current forms a current loop from power supply Vdd→Q1→first electrode→R12→second electrode→Q4→R1. At this time, the first electrode of the care chamber is positive (+), the second electrode is negative (-), the third electrode is floating, the current flows through R1, generating a divided voltage V1, which is filtered by R2 and Cl, and then enters the MCU AD sampling voltage Vad. The voltage at this port is used to determine whether it is a current loop and whether a current loop is established. If Vad has voltage, the care chamber is working normally; if Vad voltage is 0V, the care chamber is working abnormally.

If the device continuously operates in the second state or the third state, the electrodes are not switched when HCl and HClO are electrolyzed in the care chamber. If the device controls the circuit through the electrode switching module to switch between the second state and the third state, the operation mode of the device is the electrode switching mode, and the first electrode and the second electrode switch between the cathode and the anode.

Fourth state: The driving upper arm X of the MCU is at a low level (L), the driving upper arm Y is at a low level (L), the driving upper arm Z is at a low level (L), the driving lower arm U of the MCU is at a low level (L), the driving upper arm V is at a low level (L), and the driving lower arm W is at a low level (L). In this case, no current loop is formed, and the first electrode, second electrode, and third electrode in the care chamber are all in a floating state and uncharged, so the device does not operate.

Preferably, the first electrode and the second electrode are symmetrically arranged in the care chamber 1, both close to the sidewall of the care chamber 1, to achieve better protein removal effects. In the examples, the first electrode, second electrode, and third electrode are all electrode probes or electrode sheets made of chlorine-evolving materials.

In a preferred embodiment, the chloride ion-containing solution is physiological saline with a mass concentration of 0.9%; the starting voltage for connecting the first electrode and the third electrode to the power supply is not less than 1.1V, the stable voltage after connection is 3.5-6.5V, and the power supply is disconnected after the first electrode and the third electrode react for 3-11 minutes.

After the power supply to the first electrode and the third electrode is disconnected, the first electrode and the second electrode are connected to the power supply, with a starting voltage not less than 1.1V and a stable voltage of 3.5-6.5V after connection. The pH value of the solution in the care chamber is controlled within the range of 8-12, and the mass concentration of ClO⁻ in the solution is controlled at 0.1%-0.4%. The cleaning and sterilization of the contact lens are completed after the first electrode and the second electrode react for 10-60 minutes.

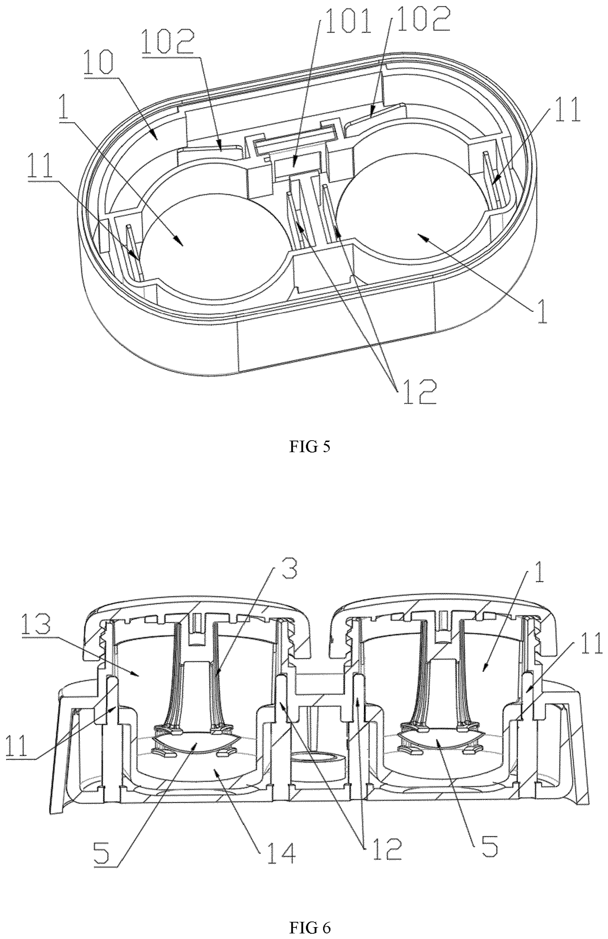

In a preferred embodiment, as shown in FIG. 6, the care chamber 1 includes an accommodation slot 13 and a sunken slot 14 connected to the accommodation slot 13. The sunken slot 14 is located below the accommodation slot 13, and the first electrode 11 and the second electrode 12 are both arranged in the accommodation slot 13.

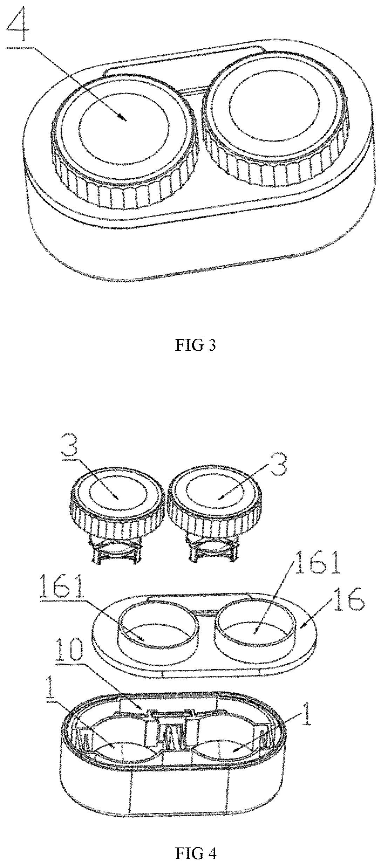

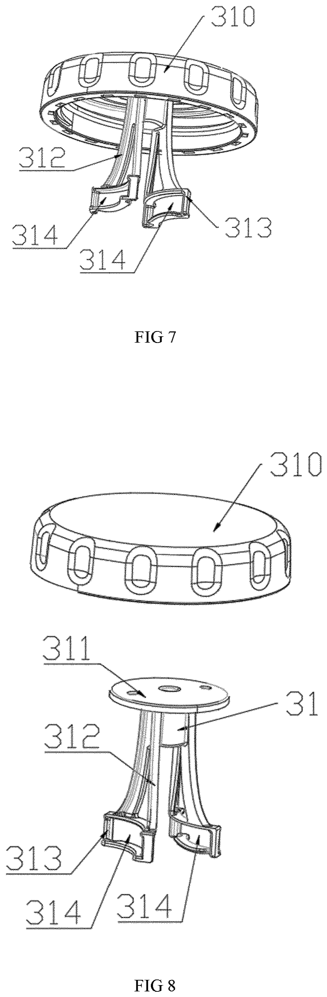

Preferably, as shown in FIG. 4 and 6-10, it also includes a lens clip 3, which can limit the contact lens to be placed in the chloride ion-containing solution in the care chamber. In one example, as shown in FIG. 7-8, the lens clip 3 is a lateral lens clamp 31. The care chamber 1 and the auxiliary chamber 10 contain the chloride ion-containing solution, and the lateral lens clamp 31 can limit the contact lens 5 to be accommodated in the sunken slot 14, with the distance between the top of the contact lens and the liquid surface of the chloride ion-containing solution being not less than 2mm. In the example, the lateral lens clamp includes a first cover 310, a mounting base 311, a support member 312, and a limit member 313. The support member 312 is vertically arranged on the mounting base 311, and the limit member 313 is provided with a limit hole 314. There are at least two limit members 313, and the contact lens is limited within the limit hole 314. During use, the length direction of the limit hole 314 is consistent with the horizontal direction. In the example, the mounting base, support member, and limit member are integrally designed, and the mounting base is detachably installed on the first cover 310.

The limit hole serves to limit the contact lens, enabling the contact lens to be horizontally accommodated in the chloride ion-containing solution during the protein removal and sterilization process, preventing movement or shaking that could affect the protein removal and sterilization efficacy or damage the contact lens. Preferably, the support member 312 is a flexible support body, facilitating the accommodation of the contact lens; the limit member 313 is a flexible limit member, preventing damage to the contact lens.

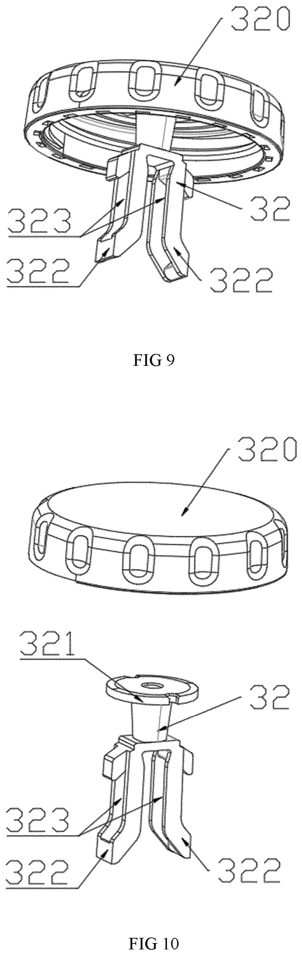

In one example, as shown in FIG. 9-10, the lens clip 3 is a vertical lens clip 32, which can position the contact lens 5 within the care chamber 1, with the plane formed by the first electrode and the second electrode arranged perpendicular to the contact lens; the distance between the contact lens 5 and the anode in the care chamber is no less than 4 mm, and the distance between the top of the contact lens 5 and the liquid surface of the chloride ion-containing solution is no less than 2 mm.

The purpose of maintaining the height of the contact lens top at no less than 2 mm from the liquid surface of the chloride ion-containing solution: during sterilization and protein removal of the contact lens, the anode can electrolyze Cl⁻ in the chloride ion-containing solution into Cl₂, which rises from near the electrode and escapes above the liquid surface. The escaped Cl₂, due to its own gravity and water solubility, dissolves in the solution to produce HCl and HClO, resulting in higher concentrations of HClO and Cl₂ near the liquid surface top. When HClO accumulates to a certain concentration, it may affect the lens color. Thus, maintaining sufficient distance between the top of the contact lens and the liquid surface can reduce the rate of discoloration of the contact lens.

The purpose of maintaining a distance of no less than 4 mm between the contact lens and the cathode probe or anode probe: during sterilization and protein removal of the contact lens, the areas near the anode and cathode are in two different pH environments. Near the cathode, H₂O in the sodium chloride solution is electrolyzed to produce H₂ and OH⁻, e+2H₂O=H₂ and OH⁻. Near the anode, Cl⁻ in the sodium chloride solution is electrolyzed into Cl₂, which dissolves in water to produce HCl and HClO, with a pH much lower than 7, creating an acidic environment. Therefore, it is necessary to maintain sufficient distance between the contact lens and the anode; otherwise, the lens color may fade easily.

As shown in FIG. 10, the vertical lens clip includes a second cover 320, a fixed base 321, and at least two accommodation members 322. The accommodation members 322 are vertically arranged on the fixed base 321, with accommodation holes 323 opened on the accommodation members 322. During use, the length direction of the accommodation holes 323 is aligned with the vertical direction. There are at least two accommodation members 322, and the contact lens is confined within the accommodation holes 323 of the accommodation members 322. In the example, the fixed base and the accommodation member are integrally designed, and the fixed base is detachably mounted on the second cover 320. The accommodation holes serve to position the contact lens, ensuring that the contact lens can be vertically accommodated in the chloride ion-containing solution during the protein removal and sterilization process, without moving or shaking, which could affect the protein removal and sterilization efficacy or damage the contact lens. Preferably, the vertical lens clip adopts an eccentric design, so that when the contact lens is placed vertically, it is biased toward the cathode, keeping the contact lens away from Cl₂ and HClO, with the contacted liquid mainly being NaClO, thereby reducing the impact on the lens color and achieving relatively better sterilization and protein removal effects. Preferably, the accommodation members 322 are flexible supports, which not only facilitate the accommodation of the contact lens but also avoid causing damage to the contact lens.

The care chamber is further provided with a cover body 16, which is closed on the care chamber 1. The cover body 16 is provided with an installation port, and the first cover 310 or the second cover 320 is closed on the installation port 161, so that the lateral lens clamp or the vertical lens clamp limits the contact lens in the chloride ion-containing solution within the care chamber.

In the example, the base 2 is internally provided with a PCB board, an energy storage component, and a control panel. The energy storage component can supply electrical energy to the device. The first electrode and the second electrode of the care chamber extend from the bottom of the care chamber to the outside of the care chamber, and the third electrode of the auxiliary chamber extends from the bottom of the auxiliary chamber to the outside of the auxiliary chamber. The base is provided with a placement groove 21 and multiple conductive contacts. The conductive contacts and the control panel are electrically connected to the PCB board. The control panel can control the sterilization and protein removal work of the care chamber through the PCB board and the control system. During the cleaning and sterilization of the contact lens, the care chamber and the auxiliary chamber are placed in the placement groove 21, and the first electrode, the second electrode, and the third electrode are respectively connected to the corresponding conductive contacts. Preferably, the care chamber and the auxiliary chamber are magnetically attracted within the placement groove 21. In the example, the care chamber and the auxiliary chamber are arranged on the same housing. Of course, the power supply can be the energy storage component or an external power supply, and this patent is not limited to this.

In the example, the base 2 is further provided with a rinsing chamber 22. After the sterilization and protein removal of the contact lens in the care chamber is completed, sterile physiological saline or multi-functional eye care solution is poured into the rinsing chamber 22. The cover body is removed, and the contact lens confined to the lens clip is transferred into the rinsing chamber 22. The rinsing chamber 22 is then activated to clean the contact lens. In the example, the rinsing chamber 22 is a vibrating rinsing chamber. Of course, other rinsing methods may also be employed, as long as they can rinse the residual liquid left on the contact lens after cleaning in the care chamber.

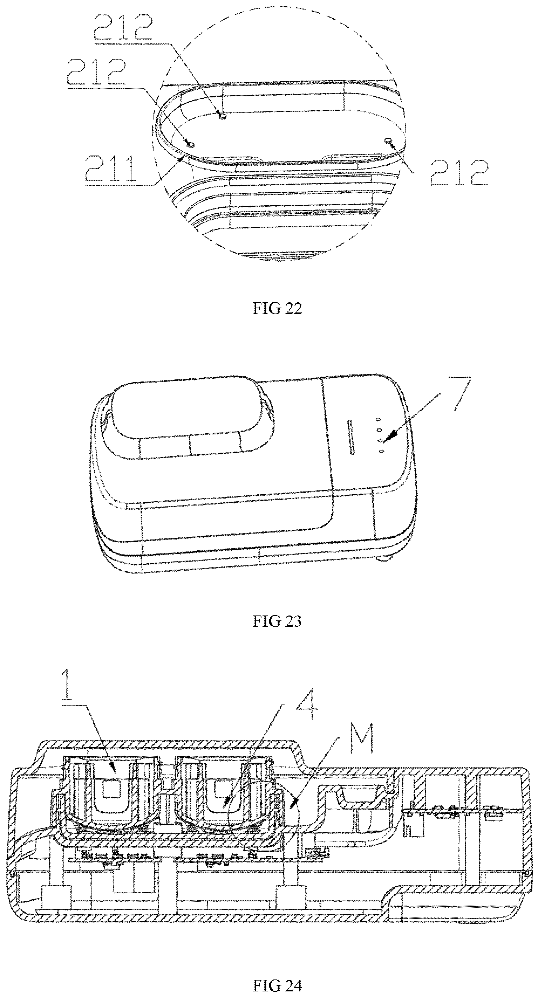

In a preferred embodiment, as shown in FIG. 12-29, the cleaning and sterilization device includes a power supply, a control switch, a care chamber 1, and an auxiliary chamber 10. The auxiliary chamber 10 is arranged around the care chamber 1, and a cation exchange membrane 101 is provided between the care chamber 1 and the auxiliary chamber 10. The cation exchange membrane 101 can prevent OH⁻ generated in the care chamber from entering the auxiliary chamber. The care chamber is provided with at least a first electrode 11 and a second electrode 12, one of which is an anode and the other is a cathode. The auxiliary chamber 10 is provided with a third electrode 102, which becomes the anode when the third electrode and the cathode in the care chamber are connected to the power supply.

The power supply can provide electrical energy to the care chamber and the auxiliary chamber, and the control switch can at least control the device to turn on or off.

Auxiliary chambers are arranged in a one-to-one correspondence around the care chambers, with each care chamber independently controlled to avoid mutual interference between different lenses and different reaction processes. This design is more aesthetically pleasing in appearance and more intuitive in operation, facilitating user interaction, preventing misuse, and providing consumers with a better and more comfortable user experience.

In the example, the side wall of the care chamber 1 is provided with a communication hole, and the communication hole is sealed with a cation exchange membrane 101.

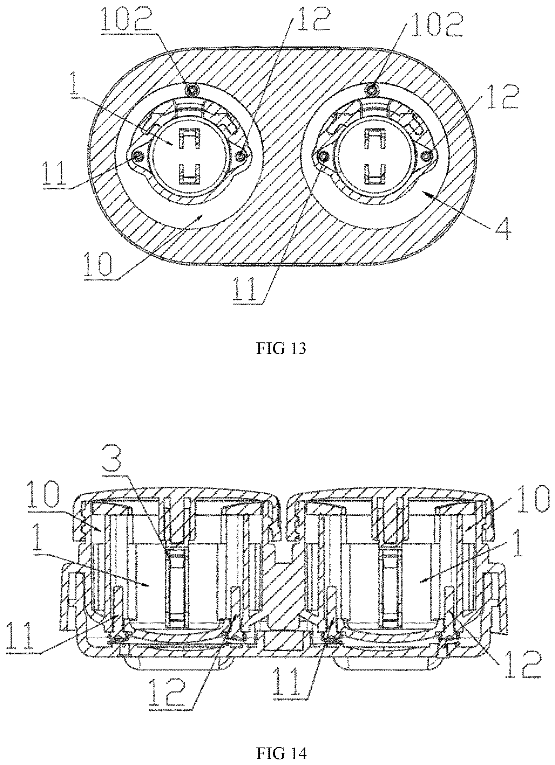

As shown in FIG. 13, the specific cleaning and sterilization steps include the following:

First, assume the first electrode 11 is the cathode (of course, the second electrode can also be initially set as the negative electrode). The care chamber contains physiological saline with a mass concentration of 0.9%. A potential difference is formed between the first electrode 11 as the cathode and the third electrode 102 as the anode, and they work for a period of time. Alkaline ions such as OH⁻ are generated near the first electrode as the cathode, making the pH value in the care chamber greater than 7. This ensures that the pH value in the care chamber is in an alkaline environment, guaranteeing that when the first electrode and the second electrode in the care chamber work subsequently, NaClO is easily produced in the care chamber due to the alkaline environment, while HClO is not easily produced, thereby reducing the adverse effects of HClO on lens discoloration and material properties. Second, the first step stops working, and a current loop is formed between the first electrode 11 and the second electrode 12, with one as the anode and the other as the cathode. They work for a certain period of time to clean and care for the contact lens.

Preferably, in the second step, the first electrode 11 remains as the cathode, and the second electrode 12 serves as the anode. Preferably, in the first step, the starting voltage for connecting the first electrode 11 and the third electrode 102 to the power supply is preferably greater than 1.1V, the stable voltage after connection is 3.5-6.5V, the power is disconnected after working for 2-11 minutes, and the pH value in the care chamber is adjusted to the range of 10-12. Further preferably, in the first step, the stable voltage after connecting the first electrode 11 and the third electrode 102 to the power supply is preferably 5V, the power is disconnected after working for 3 minutes, and the pH value in the care chamber is adjusted to the range of 10-12. Of course, the present patent is not limited thereto, and the working time and operating voltage in the first step can be selected as other values as needed.

Preferably, in the second step, the starting voltage for connecting the first electrode 11 and the second electrode 12 to the power supply is preferably greater than 1.1V, the stable voltage after connection is 3.5-6.5V, the mass concentration of ClO⁻ in the solution is controlled to be 0.01%-0.4%, and the cleaning and sterilization of the contact lens is completed after working for about 10-60 minutes. Further preferably, in the second step, the stable voltage after connecting the first electrode and the second electrode to the power supply is 5V, the mass concentration of ClO⁻ in the solution is controlled to be 0.01-0.229%, the power is disconnected after working for about 20 minutes, to achieve the best sterilization and protein removal effects, as well as the safest operating environment. Of course, the working time and operating voltage in the second step can be selected as other values as needed.

Further preferably, during the cleaning and care of the contact lens, the first electrode 11 and the second electrode 12 can undergo electrode switching, and the switching method is as described in the above technical solution.

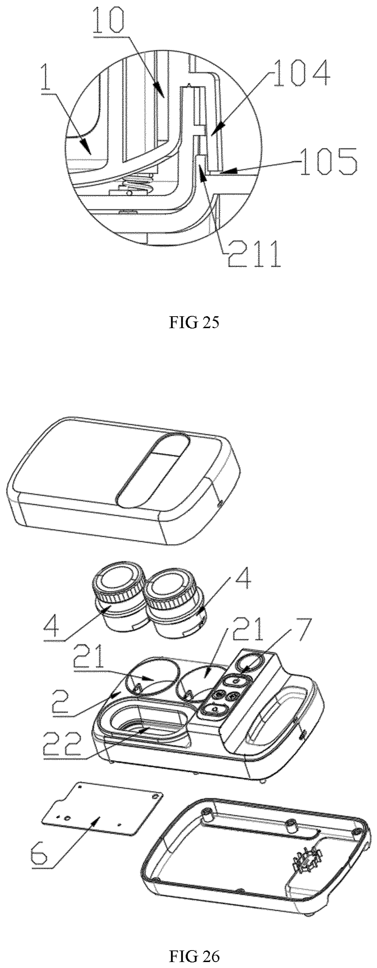

In a preferred embodiment, as shown in FIG. 26-29, the cleaning and sterilization device includes a power supply, a control switch, a care chamber 1, and an auxiliary chamber 10. A cation exchange membrane 101 is arranged between the care chamber 1 and the auxiliary chamber 10, and the cation exchange membrane 101 can prevent OH⁻ generated in the care chamber from entering the auxiliary chamber. The care chamber is provided with at least two first electrodes 11 and at least two second electrodes 12, and the auxiliary chamber 10 is provided with at least two third electrodes 102. After the third electrode is connected to the power supply with the cathode in the care chamber 1, the third electrode 102 serves as the anode.

The power supply can provide electrical energy to the care chamber and the auxiliary chamber, and the control switch can at least control the device to turn on or off.

Preferably, the quantities of the first electrode 11, the second electrode 12, and the third electrode 102 are all consistent. In the example, there are two each of the first electrode 11, the second electrode 12, and the third electrode 102. Preferably, the multiple first electrodes 11 and/or multiple second electrodes 12 are uniformly distributed near the sidewalls within the care chamber. Further preferably, the two first electrodes 11 are symmetrically arranged on both sides of the care chamber, and the two second electrodes 12 are symmetrically arranged on both sides of the care chamber. Preferably, the two first electrodes 11 are connected in series, the two second electrodes 12 are connected in series, and the two third electrodes 102 are connected in series.

Preferably, the auxiliary chamber 10 is circumferentially arranged outside the care chamber 1, with the care chamber 1 and the auxiliary chamber 10 corresponding one-to-one.

Preferably, the sidewall of the care chamber 1 is provided with a communication hole, on which a cation exchange membrane 101 is hermetically installed.

As shown in FIG. 29, the specific cleaning and sterilization steps include the following:

First step, assuming the first electrode 11 is the cathode (of course, the second electrode can also be initially set as the negative pole), the care chamber contains physiological saline with a mass concentration of 0.9%. A potential difference is formed between the two first electrodes 11 and the two third electrodes 102, operating for a period of time. The solution near the first electrode acting as the cathode generates alkaline ions such as OH⁻, causing the pH value within the care chamber to exceed 7; thereby achieving an alkaline environment within the care chamber, ensuring that during subsequent operation of the first and second electrodes within the care chamber, due to the alkaline environment, NaClO is easily produced while HClO is not easily produced, thus reducing the adverse effects of HClO on lens discoloration and material properties. Second step, the first step ceases operation, and the two first electrodes 11 and the two second electrodes 12 include the cathode and anode, forming a current loop between each other, operating for a certain period to clean and care for the contact lens.

Preferably, in the second step, the two first electrodes 11 remain as the cathode, and the two second electrodes 12 serve as the anode. Preferably, in the first step, the starting voltage between the first electrode 11 and the third electrode 102 when the power supply is connected is preferably greater than 1.1V, the stable voltage after power connection is 3.5-6.5V, the power is disconnected after working for 2-11 minutes, and the pH value in the care chamber is adjusted to the range of 10-12. Further preferably, in the first step, the stable voltage after connecting the first electrode 11 and the third electrode 102 to the power supply is preferably 5V, the power is disconnected after working for 3 minutes, and the pH value in the care chamber is adjusted to the range of 10-12. Of course, the present patent is not limited thereto, and the working time and operating voltage in the first step can be selected as other values as needed.

Preferably, in the second step, the starting voltage for connecting the first electrode 11 and the second electrode 12 to the power supply is preferably greater than 1.1V, the stable voltage after connection is 3.5-6.5V, the mass concentration of ClO⁻ in the solution is controlled to be 0.01%-0.4%, and the cleaning and sterilization of the contact lens is completed after working for about 10-60 minutes. Further preferably, in the second step, the stable voltage after connecting the first electrode and the second electrode to the power supply is 5V, the mass concentration of ClO⁻ in the solution is controlled to be 0.01-0.229%, the power is disconnected after working for about 20 minutes, to achieve the best sterilization and protein removal effects, as well as the safest operating environment. Of course, the working time and operating voltage in the second step can be selected as other values as needed.

Multiple electrodes of the same polarity are arranged in the care chamber, providing high cleaning efficiency and speed, while making NaClO, NaOH, and HClO in the care chamber more uniform, resulting in more even contact lens cleaning. A potential difference is formed internally between or mutually between the first electrode pair and the second electrode pair. Multiple electrodes of the same polarity are uniformly distributed near the sidewall inside the care chamber, eliminating the need for electrode polarity switching.

The care chamber 1 and the auxiliary chamber 10 are collectively referred to as the care assembly. The auxiliary chamber is arranged around the care chamber, making full use of space with more reasonable design, effectively reducing the volume of the care assembly.

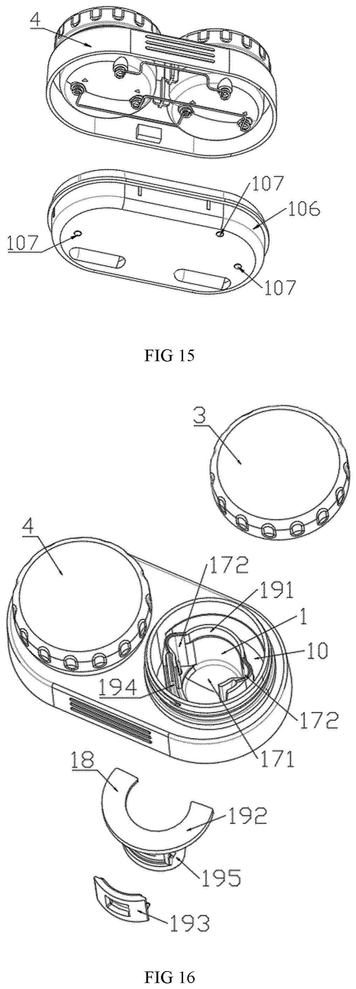

In a preferred embodiment, as shown in FIG. 16 and 27, the care chamber 1 includes a main region 171 and at least two extension regions 172. The at least two extension regions 172 are symmetrically arranged on the outer side of the main region 171, with the main region 171 and the extension regions 172 interconnected. The electrodes are disposed within the extension regions. The electrodes refer to the first electrode and the second electrode. In the example, the main region 171 is circular. Preferably, the auxiliary chamber 10 is annularly arranged around the outer side of the care chamber 1, and the care assembly is circular. By placing the electrodes in the extension regions, the distance between the electrodes can be increased without enlarging the volume of the care assembly.

In the example, as shown in FIG. 16 and 27, the first electrode is disposed in one of the extension regions 172, and the second electrode is disposed in another extension region 172. The first electrode and the second electrode are symmetrically arranged within the care chamber 1, with the two extension regions 172 symmetrically positioned on both sides of the main region 171. In the example, the first electrode and the second electrode are circular probes. Of course, this patent is not limited thereto, and the first electrode and the second electrode may also be electrode sheets or electrodes of other shapes. Of course, the first electrode and/or the second electrode may be singular or plural.

Preferably, a baffle 18 is further provided. As shown in FIGS. 16-18, 21, and 27, the baffle 18 at least shields a portion of the auxiliary chamber. In the case, the baffle 18 is sealingly connected to the top of the side wall of the care chamber and extends above the auxiliary chamber. In the case, the baffle 18 shields above the first electrode 11, the second electrode 12, and the third electrode 102. The baffle is provided to shield and protect the third electrode. The baffle also has a certain effect in preventing liquid in the care chamber from overflowing into the auxiliary chamber or liquid in the auxiliary chamber from overflowing into the care chamber. At the same time, the baffle also has a certain aesthetic appeal.

Preferably, as shown in FIGS. 16-19 and 27, the care chamber 1 includes a care chamber main body 191, a profiled connector 192, and a second connector 193. The profiled connector 192 includes the baffle 18 and a side plate 195. The baffle 18 and the side plate 195 are arranged perpendicularly. The surface of the side plate 195 is provided with a first communication hole, and the surface of the second connector 193 is provided with a second communication hole. The first communication hole and the second communication hole constitute a communication hole. The cation exchange membrane 101 is disposed between the first communication hole and the second communication hole. The second connector 193 is sealingly fixed on the surface of 195, thereby sealingly fixing the cation exchange membrane 101 between the first communication hole and the second communication hole. The side wall of the care chamber main body 191 is provided with an opening slot 194. The side plate 195 of the profiled connector 192 matches the opening slot 194. The side edge of the side plate 195 is sealingly connected to the opening slot 194. The baffle 18 is sealingly connected to the top of the side wall of the care chamber 1 and shields above the first electrode, the second electrode, and the cation exchange membrane. In the case, the side plate is an arc-shaped plate matching the side wall of the care chamber. The second connector is sealingly connected to the side plate of the profiled connector, and the profiled connector is sealingly connected to the care chamber main body to prevent leakage points between the care chamber and the auxiliary chamber, which may affect the process control during care and further impact the cleaning effect of care.

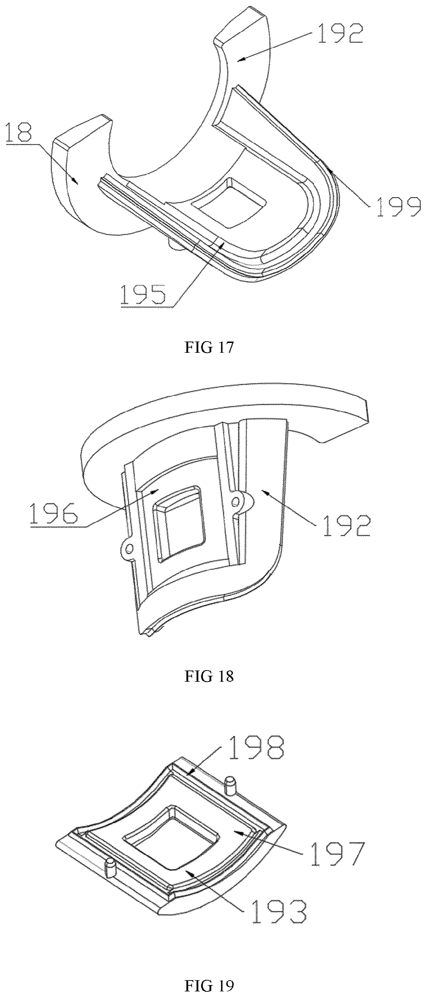

Preferably, as shown in FIG. 18-19, the side plate 195 is provided with a mounting groove 196 on its surface, the mounting groove 196 corresponds to the first communication hole, the cation exchange membrane is accommodated in the mounting groove 196, the second connector is provided with a pressing block 197 matching the mounting groove 196, and the pressing block 197 presses the cation exchange membrane in the mounting groove 196. Further preferably, the second connector 193 is provided with an annular flange 198, the annular flange 198 is arranged around the outside of the pressing block 197, and the annular flange 198 is ultrasonically welded to fix the second connector to the side plate 195. In the example, the side wall of the side plate 195 facing the opening groove is provided with a second flange 199, and the side plate 195 is ultrasonically welded to the opening groove via the second flange 199. The top of the care chamber is provided with a third flange at a position corresponding to the baffle, and the baffle is ultrasonically welded to the top of the care chamber via the third flange. Preferably, as shown in FIG. 17-18, the opening groove 194 is an arc-shaped groove. The opening groove is designed as an arc-shaped groove, which provides better sealing through ultrasonic welding without leakage points. In the example, further, the second connector is provided with a recess, the side plate is provided with a mounting post, the mounting post is accommodated in the recess, and the second connector is fixed to the arc-shaped plate to provide additional positioning and fixation.

In the example, the main body of the care chamber and the auxiliary chamber are integrally designed.

In the example, the auxiliary chamber is an annular auxiliary chamber arranged around the outside of the care chamber. Of course, the present patent is not limited to this, and the auxiliary chamber may also be an arc-shaped auxiliary chamber arranged around the outside of the care chamber or others.

In one case, the two care assemblies are integrally formed to constitute a care part, the corresponding electrodes in the two care assemblies are connected in series respectively, and the left-eye contact lens and the right-eye contact lens are placed in the corresponding care assemblies for simultaneous care and cleaning. As shown in FIG. 12-16, the care part includes two integrally formed care assemblies, the two care chambers are integrally designed, each care chamber is surrounded by an auxiliary chamber, each care chamber is connected to each auxiliary chamber through a cation exchange membrane, the two care chambers are independent of each other, and the two auxiliary chambers are also independent of each other. In another case, the two care assemblies are independent of each other, constituting the care part. As shown in FIG. 26-29, the care part includes two care assemblies with a split design, each care assembly includes a care chamber and an auxiliary chamber surrounding the care chamber, and the care chamber and the auxiliary chamber are connected through a cation exchange membrane. Certainly, in other cases, to further reduce the volume or for portability, the care part includes only one care assembly, and the left-eye contact lens and the right-eye contact lens are cleaned and cared for in sequence. Alternatively, to save time, the care part may also include more than two care assemblies, etc., and the number of care assemblies can be flexibly adjusted as needed.

The first electrode, the second electrode, the third electrode, etc., arranged in the care chamber or the cathode or anode in the auxiliary chamber are collectively referred to as electrodes.

Preferably, the bottom of the care assembly further includes a bottom cover 106, the bottom cover 106 is provided with a plurality of second electrode contacts 107, the bottom of the electrode extends outside the care chamber or the auxiliary chamber, as shown in FIG. 14-15 and FIG. 27, the electrodes of the same polarity in the two care chambers (e.g., the same first electrode, the same second electrode; or the same cathode, the same anode) are connected in series at the bottom through wires, the electrodes of the same polarity in the two auxiliary chambers are connected in series at the bottom through wires, and the same electrodes are connected in series and then electrically connected to the corresponding second electrode contacts. In the case, the end of the wire is wound into a spiral spring, the spiral spring is electrically sleeved at the bottom of the electrode, and the second electrode contact is electrically connected to the corresponding electrode bottom through the spiral spring. Or electrodes of the same polarity within the same care chamber are connected in series via wires, and then electrically connected to the corresponding second electrode contact. Of course, electrodes of the same polarity can also be connected in series via a circuit board and then electrically connected to the second electrode contact, as shown in FIG. 27. Using a spring to connect with the second electrode contact facilitates assembly, provides elastic contact that is less prone to loosening, and does not require high assembly precision. Additionally, after electrodes of the same polarity within the same care chamber or auxiliary chamber are connected in series via wires or a circuit board, one of the electrodes of the same polarity can also be electrically connected to the corresponding second electrode contact by cold pressing. Preferably, a recessed groove is provided at the bottom of the electrode, and the corresponding second electrode contact is cold-pressed into the recessed groove. Using cold pressing ensures full contact between the electrode and the second electrode contact, enhancing conductivity.

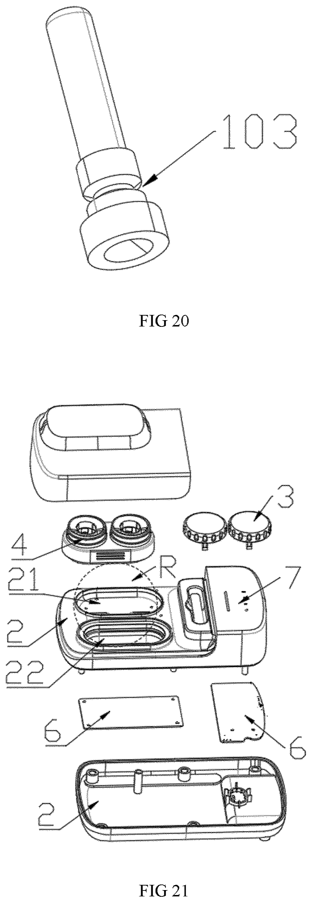

Preferably, as shown in FIG. 14 and 20, a limit structure is provided near the bottom end of the electrode, and the limit structure is fixedly accommodated at the bottom of the care chamber or auxiliary chamber. For the care assembly, during its preparation, the electrode is pre-placed in the mold, and then the care assembly is injection-molded, with the limit structure accommodated within the care assembly. The electrodes are equipped with limiting structures, which not only ensure sealed connection between the electrodes and the care assembly to prevent liquid leakage, but also firmly install the electrodes on the care assembly, reducing the likelihood of loosening or shaking during device assembly or use. In the example, the limit structure is a limit groove 103, preferably, the limit groove is an annular limit groove. Of course, the present patent is not limited to this, and may also include limit protrusions or others, as long as they can serve the limiting function.

Preferably, the cleaning and sterilization device further includes a base 2 and a control system, wherein the base 2 is provided with a placement groove 21, and the care assembly is placed in the placement groove. Further preferably, the base 2 is also provided with a rinsing chamber 22, which is configured to rinse residual liquid from the contact lens after cleaning and care in the care chamber. The specific method of use is as follows: pour sterile physiological saline or multi-functional eye-safe care solution into the rinsing chamber 22, take the lens clip holding the contact lens, transfer the contact lens fixed on the lens clip into the rinsing chamber 22, activate the rinsing chamber 22, and clean the contact lens. Preferably, the care assembly is magnetically placed in the placement groove 21.

In a preferred embodiment, during use, the care assembly is placed in the placement groove 21. Preferably, as shown in FIG. 14 and 24-25, a surrounding edge 211 is provided around the placement groove 21. The surrounding edge is provided to prevent water on the base from flowing into the placement groove, thereby corroding the conductive contacts in the placement groove or causing short circuits between the conductive contacts, which would affect the cleaning and care effectiveness of the contact lens in the care chamber. Preferably, the care assembly is magnetically attached in the placement groove 21.

Further preferably, the care assembly is provided with a cover edge 104 that matches the surrounding edge, wherein the cover edge 104 is arranged outside the surrounding edge 211, and a gap 105 is provided between the cover edge 104 and the base. A gap is provided to prevent liquid on the base from entering the placement groove through capillary action, thereby corroding the conductive contacts within the placement groove or causing a short circuit between the conductive contacts. Of course, in other embodiments, as shown in FIG. 26-28, the care assembly can also be snapped into the placement groove.

In a preferred embodiment, a flexible net (not shown in the figure) is additionally arranged within the rinsing chamber 22, and the lens clip holding the contact lens is placed within the flexible net. Preferably, the shape of the flexible net matches the outer contour of the lens clip holding the contact lens. The flexible net is placed within the rinsing chamber, and the lens clip holding the contact lens places the contact lens into the rinsing chamber. The rinsing mode is then activated to rinse the contact lens. After rinsing is completed, the contact lens can be worn directly. Preferably, an ultrasonic component is arranged at the bottom of the rinsing chamber 22. The ultrasonic vibration mode is used to rinse the contact lens, thereby removing residual liquid from the contact lens during care. Preferably, the flexible net is detachably placed within the rinsing chamber. This facilitates periodic or non-periodic thorough cleaning or sterilization of the flexible net. By setting up the flexible net, during ultrasonic vibration rinsing of the contact lens, the flexible net can physically clean the surface of the contact lens, ensuring a cleaner and more thorough surface rinse.

The base 2 is internally provided with a PCB board 6 and a control panel 7. The first electrode and the second electrode of the care chamber extend from the bottom of the care chamber to the outside of the care chamber. The third electrode of the auxiliary chamber extends from the bottom of the auxiliary chamber to the outside of the auxiliary chamber. The base is provided with a placement groove 21 and multiple conductive contacts 212. The conductive contacts 212 and the control panel 7 are electrically connected to the PCB board 6. The control panel 7 can control the cleaning and sterilization work of the care chamber through the PCB board 6 and the control system. Preferably, the base may be internally provided with an energy storage component, which supplies power to the device. Of course, the device may also be provided with an external cable to supply power to the device by connecting to an external power supply.

All features of the above components can be freely combined without conflict. In addition, structural changes, modifications, and variations of the components are also within the scope of protection of this patent.

In the description of this specification, references to terms such as "one embodiment," "another embodiment," "yet another embodiment," "other embodiments," "example," or "specific example" mean that the specific features, structures, materials, or characteristics described in connection with the embodiment or example are included in at least one embodiment or example of the present invention. In this specification, the schematic descriptions of the above terms do not necessarily refer to the same embodiment or example. Moreover, the described specific features, structures, materials, or characteristics may be combined in any suitable manner in one or more embodiments or examples. Furthermore, those skilled in the art can combine and integrate the different embodiments or examples described in this specification.

Although the embodiments of the present invention have been shown and described above, it is to be understood that the above embodiments are exemplary and should not be construed as limiting the present invention. Those of ordinary skill in the art may make variations, modifications, and adaptations to the above embodiments within the scope of the present invention.

## Claims

1. A method for cleaning and sterilizing contact lens using electrochemistry, **characterized by** comprising the following steps:
S1: pouring a chloride ion-containing solution into a care chamber and an auxiliary chamber, which are interconnected, wherein a cation exchange membrane is placed between the care chamber and the auxiliary chamber, the care chamber is equipped with at least a first electrode and a second electrode, while the auxiliary chamber is equipped with a third electrode, the contact lens is placed in the chloride ion-containing solution within the care chamber;
S2: connecting the first electrode and the third electrode to a power supply, wherein the first electrode serves as a cathode, the third electrode serves as an anode, a reduction reaction occurs at the first electrode, and the first electrode electrolyzes H₂O in the chloride ion-containing solution in the care chamber to produce H₂ and OH⁻; an oxidation reaction occurs at the third electrode, and the third electrode electrolyzes Cl⁻ in the chloride ion-containing solution in the auxiliary chamber to produce Cl₂, which dissolves in the solution to form HCl and HClO, and the cation exchange membrane can block Cl⁻ and ClO⁻ in the auxiliary chamber from entering the care chamber;
S3: when the pH in the care chamber reaches 8-12, disconnecting the power to the first and third electrodes and connecting the first and second electrodes to the power source, with one serving as the anode and the other as the cathode, the cathode electrolyzes H₂O in the solution to produce H₂ and OH⁻, while the anode electrolyzes Cl⁻ into Cl₂, which dissolves to form Cl⁻ and ClO⁻, the ClO⁻ sterilizes the contact lens and degrades denatured proteins on it, the degraded proteins move toward the oppositely charged electrode under the electric field formed between the anode and cathode, achieving sterilization and protein removal.

2. The method for cleaning and sterilizing contact lens using electrochemistry according to claim 1, **characterized in that** the chloride ion-containing solution is an NaCl solution with a pH value of 7;
the first electrode, second electrode, and third electrode are all chlorine evolution electrodes.

3. The method for cleaning and sterilizing contact lens using electrochemistry according to claim 1, **characterized in that** in step S3, after the first electrode and the second electrode are connected to the power supply, the first electrode and the second electrode switch between the cathode and the anode at certain time intervals.

4. The method for cleaning and sterilizing contact lens using electrochemistry according to claim 2, **characterized in that** the NaCl solution is a physiological saline with a mass concentration of 0.9%;
in step S2, the power supply of the first electrode and the third electrode is disconnected after 3-11 minutes of reaction;
in step S3, the pH value of the solution in the care chamber is controlled to be within the range of 8-12, the mass concentration of ClO⁻ in the solution is controlled to be 0.1%-0.4%, and the cleaning and sterilization of the contact lens are completed after 10-60 minutes of reaction between the first electrode and the second electrode.

5. The method for cleaning and sterilizing contact lens using electrochemistry according to claim 2, **characterized in that** in step S3, the bottom heights of the first electrode and the second electrode are both higher than the bottom height of the care chamber, the contact lens is horizontally placed in the chloride ion-containing solution in the care chamber, the top height of the contact lens is lower than the bottom height of the probe, and the distance between the top of the contact lens and the liquid surface of the chloride ion-containing solution is not less than 2 mm; or
the contact lens is placed vertically in the chlorine ion-containing solution in the care chamber, the distance from the contact lens to the anode is not less than 4mm, and the distance from the contact lens to the liquid surface of the chlorine ion-containing solution is not less than 2mm.

6. A method for cleaning and sterilizing contact lens using electrochemistry, **characterized by** comprising the following steps:
S1: pouring a chloride ion-containing solution into the care chamber and auxiliary chamber, which are interconnected, wherein a cation exchange membrane is placed between the care chamber and auxiliary chamber, the care chamber is equipped with at least a first electrode and a second electrode, while the auxiliary chamber is equipped with a third electrode, and the contact lens is placed in the chloride ion-containing solution within the care chamber;
S2: connecting the first electrode and the third electrode to the power supply, wherein the first electrode serves as the cathode and the third electrode as the anode, the third electrode undergoes an oxidation reaction, electrolyzing Cl⁻ in the chloride ion-containing solution in the auxiliary chamber into Cl₂, which dissolves in the solution to produce HCl and HClO, the first electrode undergoes a reduction reaction, electrolyzing H₂O in the chloride ion-containing solution in the care chamber to produce H₂ and OH⁻, the cation exchange membrane prevents OH⁻ generated in the care chamber from entering the auxiliary chamber, maintaining the pH environment in the care chamber;
S3: when the pH in the care chamber reaches 8-12, disconnecting the power to the first and third electrodes and connecting the first and second electrodes to the power source, with one serving as the anode and the other as the cathode, the cathode electrolyzes H₂O in the solution to produce H₂ and OH⁻, while the anode electrolyzes Cl⁻ into Cl₂, which dissolves to form Cl⁻ and ClO⁻, the ClO⁻ sterilizes the contact lens and degrades denatured proteins on it, the degraded proteins move toward the oppositely charged electrode under the electric field formed between the anode and cathode, achieving sterilization and protein removal.

7. A device for cleaning and sterilizing contact lenses using electrochemistry, comprising a power supply and a control switch, **characterized by** comprising a care chamber (1) and an auxiliary chamber (10), which are interconnected, with a cation exchange membrane (101) arranged between them, the cation exchange membrane (101) prevents OH⁻ generated in the care chamber from entering the auxiliary chamber,
the care chamber (1) is equipped with at least a first electrode (11) and a second electrode (12), when connected to the power supply, one of the first electrode (11) and the second electrode (12) serves as the anode, and the other as the cathode, the auxiliary chamber (10) contains a third electrode, which serves as the anode.

8. The device for cleaning and sterilizing contact lens using electrochemistry according to claim 7, **characterized in that**, under the working condition of adding chloride ion-containing solution to the care chamber, the anode undergoes an oxidation reaction, and the cathode undergoes a reduction reaction, the cathode electrolyzes H₂O in the chloride ion-containing solution to produce H₂ and OH⁻, while the anode electrolyzes Cl⁻ in the chloride ion-containing solution into Cl₂, which dissolves to produce HCl and HClO, the ClO⁻ sterilizes the contact lens and degrades denatured proteins on it, the degraded denatured proteins move toward the electrode with the opposite charge under the electric field force formed between the anode and cathode, achieving sterilization and protein removal for the contact lens.

9. The device for cleaning and sterilizing contact lens using electrochemistry according to claim 7, **characterized in that** there are two care chambers (1), both interconnected with the auxiliary chamber (10), or each care chamber (1) is interconnected with its corresponding auxiliary chamber (10).

10. The device for cleaning and sterilizing contact lens using electrochemistry according to claim 7, **characterized in that** the care chamber (1) comprises an accommodation slot (13) and a sunken slot (14) interconnected with it. The sunken slot (14) is located below the accommodation slot (13), and the first electrode (11) and the second electrode (12) are both arranged within the accommodation slot (13).

11. The device for cleaning and sterilizing contact lens using electrochemistry according to claim 10, **characterized in that** it further comprises a lateral lens clamp, wherein the care chamber (1) and the auxiliary chamber (10) contain a chloride ion-containing solution, and the lateral lens clamp is capable of positioning the contact lens (5) within the sunken slot (14), with the distance between the top of the contact lens and the liquid surface of the chloride ion-containing solution being not less than 2mm; or
further comprises a vertical lens clip, wherein the vertical lens clip is capable of positioning the contact lens (5) within the care chamber (1); the distance between the contact lens (5) and the anode in the care chamber is not less than 4mm, and the distance between the top of the contact lens (5) and the liquid surface of the chloride ion-containing solution is not less than 2mm.

12. The device for cleaning and sterilizing contact lens using electrochemistry according to claim 7, **characterized in that** it further comprises a control system, wherein the control system is provided with an electrode switching module, and the electrode switching module enables the first electrode and the second electrode to switch between the cathode and the anode; and/or
the first electrode and the second electrode are symmetrically arranged within the care chamber (1), and both the first electrode and the second electrode are close to the side wall of the care chamber (1).

13. A device for cleaning and sterilizing contact lens using electrochemistry, comprising a power supply and a control switch, **characterized by** comprising a care chamber (1) and an auxiliary chamber (10), wherein the auxiliary chamber (10) is arranged around the care chamber (1), and a cation exchange membrane (101) is provided between the care chamber (1) and the auxiliary chamber (10), and the cation exchange membrane (101) is capable of blocking OH⁻ generated in the care chamber from entering the auxiliary chamber;
the care chamber (1) is provided with at least a first electrode (11) and a second electrode (12), wherein after the first electrode (11) and the second electrode (12) are connected to the power supply, one of the first electrode (11) and the second electrode (12) serves as an anode and the other as a cathode, and the auxiliary chamber (10) is provided with a third electrode, wherein after the third electrode is connected to the power supply with the cathode in the care chamber (1), the third electrode serves as an anode.

14. A device for cleaning and sterilizing contact lens using electrochemistry, comprising a power supply and a control switch, **characterized by** further comprising a care chamber (1) and an auxiliary chamber (10), wherein a cation exchange membrane (101) is provided between the care chamber (1) and the auxiliary chamber (10), and the cation exchange membrane (101) is capable of blocking OH⁻ generated in the care chamber from entering the auxiliary chamber;
the care chamber (1) is provided with multiple first electrodes (11) and multiple second electrodes (12), wherein after the first electrodes (11) and the second electrodes (12) are connected to the power supply, one of the first electrodes (11) and the second electrodes (12) serves as an anode and the other as a cathode;
the auxiliary chamber is provided with multiple third electrodes (102), wherein after the third electrodes are connected to the power supply with the cathode in the care chamber (1), the third electrodes serve as anodes.

15. The device for cleaning and sterilizing contact lens using electrochemistry according to any one of claims 7, 13, and 14, **characterized in that** a limit structure is provided at the end of the first electrode (11) and/or the second electrode (12) and/or the third electrode (102) near the bottom, and the limit structure is fixedly positioned at the bottom of the care chamber or the auxiliary chamber.

16. The device for cleaning and sterilizing contact lens using electrochemical technology according to any one of claims 7, 13, and 14, **characterized in that** it further comprises a baffle (18), wherein the baffle (18) at least partially covers the auxiliary chamber; and/or
the care chamber (1) and the auxiliary chamber constitute a care assembly, and the care assembly is circular.

17. The device for cleaning and sterilizing contact lens using electrochemical technology according to any one of claims 7, 13, and 14, **characterized in that** it further comprises a base (2), wherein the base (2) is provided with a placement groove (21) and a rinsing chamber (22), the care chamber and the auxiliary chamber are combined to form the care assembly, and the care assembly is placed in the placement groove;
the rinsing chamber is configured to rinse residual liquid from the contact lenses cleaned and cared for in the care chamber; the rinsing chamber (22) is further provided with a flexible net, and a lens clip holding the contact lenses is placed within the flexible net.
